# EUROPEAN PATENT SPECIFICATION

(11) **EP 2 726 617 B1**
(45) Date of publication and mention of the grant of the patent: **16.01.2019**
(21) Application number: 12737397.5
(22) Date of filing: 29.06.2012
(51) Int. Cl.: C12N 15/82, C07K 14/325

(54) **AXMI277 NEMATODE TOXIN AND METHODS FOR ITS USE**
GEGEN NEMATODEN WIRKSAMES TOXIN AXMI277 UND VERWENDUNGSMETHODEN
TOXINE AXMI277 ACTIVE CONTRE DES NÉMATODES ET PROCÉDÉS D'UTILISATION

(30) Priority: 30.06.2011 US 201161503132 P
(43) Date of publication of application: 07.05.2014
(73) Proprietor: Athenix Corp., Morrisville, NC 27560 (US)
(72) Inventor: POUTRE, Candace, Moncure, NC 27559 (US); KAHN, Theodore, W., Apex, NC 27523 (US); ANTHONY, Alissa, Morrisville, NC 27560 (US)
(74) Representative: BASF IP Association
(86) International application number: PCT/US2012/044751
(87) International publication number: WO 2014/003769

(56) References cited:
- WO-A2-2007/147029
- WO-A2-2010/027808
- US-A1- 2010 226 951
- WEI J-Z ET AL: "BACILLUS THURINGIENSIS CRYSTAL PROTEINS THAT TARGET NEMATODES", PROCEEDINGS OF THE NATIONAL ACADEMY OF SCIENCES, NATIONAL ACADEMY OF SCIENCES, US, vol. 100, no. 5, 4 March 2003 (2003-03-04) , pages 2760-2765, XP009084270, ISSN: 0027-8424, DOI: 10.1073/PNAS.0538072100
- SARVJEET KAUR: "Molecular approaches for identification and construction of novel insecticidal genes for crop protection", WORLD JOURNAL OF MICROBIOLOGY AND BIOTECHNOLOGY, KLUWER ACADEMIC PUBLISHERS, DO, vol. 22, no. 3, 1 March 2006 (2006-03-01), pages 233-253, XP019271780, ISSN: 1573-0972

### SEQUENCE LISTING

<110> Poutre, Candace Kahn, Theodore Anthony, Alissa
<120> AXMI277 NEMATODE TOXIN AND METHODS FOR ITS USE
<130> APA116020US
<150> 61/503,132
   <151> 2011-06-30
<160> 4
<170> PatentIn version 3.5
<210> 1
   <211> 3967
   <212> DNA
   <213> Bacillus thuringiensis
<400> 1
   atggattgta atttacgatc gcaacaaaat attccatata atgtattagc aacacaagca      60
   tctaatctta gtcagtttac tgatatagct gaaggtgtaa aaaaagcatg ggcagaattt     120
   caaaaaactg gatctttttc attagaagct ttaaaacaag gatttaatgc agcacaggga     180
   ggaacattca attatttagc attactacaa tcaggaatat cattagctgg ttcttttgtc     240
   cctggaggtt cttttgtagc acccattgtt aatatggtta ttggttggtt atggccaaat     300
   aaaaacaaaa cagcggatac agaaaattta ataaaattaa ttgatgaaga aattcaaaaa     360
   caattaaaca aagccttatt agaacaagac aaaaacaatt ggacctcttt tttagaaagt     420
   atatttgatg tttcaaatac agtgaataat gcaattatag atgcacagtg gtcaggtact     480
   gtagatgata caaatagaca actaaaaact ccaacaacat cagattataa aaatgttgtt     540
   gaaaaatttg attcagcgga tactgcaatt ataactaatg aaaatcaaat aatgaacggc     600
   aactttgacg tagctgcatc atcctatttt gttataggag caacattacg tcttgcatta     660
   tttcaatctt atattaaatt ttgtaatcat tggattgata cagttggatt tgattcagat     720
   aattataata cacaaaaggc taatttagct cgtacaaaac aaactatgcg tactacaatt     780
   aatgattata cacaaaaaat tatgaaagtt tttaaaaatt ccgacaatat gcctacaatg     840
   ggtactaata aatttagtgt tgatgcttat aatgcatata ttaaaggaat aacattaaat     900
   gttttagata tagtatcaac gtggccctca ttatatccaa atgattatac ttcacaaaca     960
   aagttagaac aaacacgtat cattttttca aatatgattg gacaacaaga agctatagat     1020
   ggaaccgtaa caatttacga tacttttgat tctgataatt ataaacataa accaatacct    1080
   aataataatg ttaatttact ttcttatttt actgatgaat tacaaaatat acaactcgca    1140
   ctatatacag ctcctcctaa acacaaaagt gatactcggg atagctatac gtatccttat    1200
   ggatttattt taaattacca aaatagcaaa tataaatatg gcgataacga accagtgatt    1260
   tcaaacacaa tatctgcacc tatacaacaa attaatgcag caactcaata cactcaatat 1320
   atagatggag aaagtatcaa tggcattggc gcatatttac ctggttattg ttctacagat 1380
   tgttcagaaa taactcctcc ttttgcttgc acttctaacg ataaaaataa gagctatgga 1440
   gcaagctgta atagcgtata ttctagtcaa aaaatgaatg ctttatatcc ttttacacaa 1500
   actaatgtac caggaaacca ggggaaatta ggagtactgg caagttatgt tccatatgat 1560
   ttaaatccta aaaatatatt tggtgaagta gatccagata caaataatat tatcttaaaa 1620
   ggaattcctg cagaaaaagg ctatttttct aattatacgc gacctactgt tgtaaaagaa 1680
   tggattaatg gtgcaaatgc tgtatcactt tattcaggaa atactttatt tatagtcgct 1740
   acgaatataa cagctactca atataaaatt agaatacgtt atgcaaatcc aaattcagat 1800
   actgaaatca gtgtacaaat tacacaaaat aattctctat tacacagtga tacaataaca 1860
   tttcatagta ctactgattc aaatatgaat aataatttat cacaaaatgt atatgttaca 1920
   ggggaaaatg gaaattatac acttctagat ttatatgata ctactaatgt tttatcaaca 1980
   ggagatatta cattacaaat tacaggagga agtcaagaaa tatttattga tcgaatagaa 2040
   tttattccta ctgcgcctgc gcctgctcct actaacgaca ataacaatcc ccctttccac 2100
   ggttgtttaa tagctggtga acaacaactt tgttctggac cacctaaatt tgaacaatta 2160
   agtgatttag aaaaaattac aacacaagta tatatgttat tcaaatcttc ttcatatgaa 2220
   gaattagatc caaaagtttc tagctatcaa attaatcaag tcgcattgaa agttatgtca 2280
   ctatctgatg aaatgttttg tgaagaaaaa agattgttac gaaaattagt caataaagca 2340
   aaacagttag tagaagcacg taacttacta gtaggtggaa gttttgatac acttcaaaat 2400
   tggttacttg gaacaaatgc tactataaat tatgattcgt ttttatttaa tggaaattat 2460
   ttattcttac aaccagcaag tggatttttc tcatcttatg cttatcaaaa aataaatgag 2520
   tcaaaattaa aatcatatac acgatataaa gtttctggat tcattggaca aagtaatcaa 2580
   gtagaactta ttatttctcg ttatggaaaa gaaattaata aaatattaaa tatttcatat 2640
   gcagggcctc ttcctattac ttctaataca tcaacaactt gttgtgcacc aaatataggt 2700
   caatgtaatg aagagcaatc taattctcat ttcttcagct atagcatcga tgtaggtgaa 2760
   ctttaccccg aattaaatcc tggcattgaa tttggtcttc gtattgtgga accaaatagt 2820
   tatatgacaa ttagtaattt agaaattatt gaagaacgtt cacttacaga aatggaaatt 2880
   caaacaatca aacgaaaaga tcaaaaatgg aaaaaagaaa tacttcaaga gtgtgcaaat 2940
   attaacgaac ttttacaacc aattatagat aaagtcgatt cattattcaa agatgccgac 3000
   tggtatggtc agattcttcc tcatatcaca tatcaaaatc taaaaaatat tgtattacct 3060
   gaattaccta aattgagaca ttggtttata aacgatcttg caggtgaata ttatgaaatt 3120
   gaacaaaaga tccagcaagc tctaaaacat gcatttagac aattagacga aagaaattta 3180
   atccacaacg gtcactttac agctaactta atagattggc aaacagaagg taatgcccaa 3240
   atgaaaatat tagaaaatgg tgctctcgca gcacaactct tgtcttggga ttctagtatt 3300
   tcacaatctt taaatatatt agactttgat gaggataaag catataaact tcgtgtatat 3360
   gctcaaggaa gcggaacaat ccaatttaaa aactgtgaag atgaaaccat ccaatttaat 3420
   acaaactcat tcacatataa agaaaaaata ttctatttcg atactccatc aattaactta 3480
   caaatacaat cagaaggttc taatttcgtt ataagtagta tcgagctcat tgaattatca 3540
   gcagacgaat aaattataaa aaaatggcca tatgctaaat agcatatggc catttttttt 3600
   tataaatttc accatacata ctcattggtt ctgttgcaaa gtttgaaaaa acataaatcc 3660
   gtttacaaaa tacgattatc gtttaagaaa ttttgaataa ttgctgtaat tatttgtata 3720
   tagcatgaat catctcaatt tctttcaatg tacataaagc atgacggaaa ttttgaaatc 3780
   ctgcggattt aacaaaacgt ctcttgatat atctacaatc ttgttcaata aaattattga 3840
   aatgcttaac gatacaatgt ttcgtatgca tataaagtcc attatggttc agtgttatga 3900
   gtgcataaag tagagcggga gccttatcta ttgtgagaat tgtcagttct ccaaaaactt 3960
   ttactaa 3967
<210> 2
   <211> 1183
   <212> **PRT**
   <213> Bacillus thuringiensis
<400> 2
   Met Asp Cys Asn Leu Arg Ser Gln Gln Asn Ile Pro Tyr Asn Val Leu
   1               5                   10                  15
   Ala Thr Gln Ala Ser Asn Leu Ser Gln Phe Thr Asp Ile Ala Glu Gly
               20                  25                  30
   Val Lys Lys Ala Trp Ala Glu Phe Gln Lys Thr Gly Ser Phe Ser Leu
           35                  40                  45
   Glu Ala Leu Lys Gln Gly Phe Asn Ala Ala Gln Gly Gly Thr Phe Asn
       50                  55                  60
   Tyr Leu Ala Leu Leu Gln Ser Gly Ile Ser Leu Ala Gly Ser Phe Val
   65                  70                  75                  80
   Pro Gly Gly Ser Phe Val Ala Pro Ile Val Asn Met Val Ile Gly Trp
                   85                  90                  95
   Leu Trp Pro Asn Lys Asn Lys Thr Ala Asp Thr Glu Asn Leu Ile Lys
               100                 105                 110
   Leu Ile Asp Glu Glu Ile Gln Lys Gln Leu Asn Lys Ala Leu Leu Glu
           115                 120                 125
   Gln Asp Lys Asn Asn Trp Thr Ser Phe Leu Glu Ser Ile Phe Asp Val
       130                 135                 140
   Ser Asn Thr Val Asn Asn Ala Ile Ile Asp Ala Gln Trp Ser Gly Thr
   145                 150                 155                 160
   Val Asp Asp Thr Asn Arg Gln Leu Lys Thr Pro Thr Thr Ser Asp Tyr
                   165                 170                 175
   Lys Asn Val Val Glu Lys Phe Asp Ser Ala Asp Thr Ala Ile Ile Thr
               180                 185                 190
   Asn Glu Asn Gln Ile Met Asn Gly Asn Phe Asp Val Ala Ala Ser Ser
           195                 200                 205
   Tyr Phe Val Ile Gly Ala Thr Leu Arg Leu Ala Leu Phe Gln Ser Tyr
       210                 215                 220
   Ile Lys Phe Cys Asn His Trp Ile Asp Thr Val Gly Phe Asp Ser Asp
   225                 230                 235                 240
   Asn Tyr Asn Thr Gln Lys Ala Asn Leu Ala Arg Thr Lys Gln Thr Met
                   245                 250                 255
   Arg Thr Thr Ile Asn Asp Tyr Thr Gln Lys Ile Met Lys Val Phe Lys
               260                 265                 270
   Asn Ser Asp Asn Met Pro Thr Met Gly Thr Asn Lys Phe Ser Val Asp
           275                 280                 285
   Ala Tyr Asn Ala Tyr Ile Lys Gly Ile Thr Leu Asn Val Leu Asp Ile
       290                 295                 300
   Val Ser Thr Trp Pro Ser Leu Tyr Pro Asn Asp Tyr Thr Ser Gln Thr
   305                 310                 315                 320
   Lys Leu Glu Gln Thr Arg Ile Ile Phe Ser Asn Met Ile Gly Gln Gln
                   325                 330                 335
   Glu Ala Ile Asp Gly Thr Val Thr Ile Tyr Asp Thr Phe Asp Ser Asp
               340                 345                 350
   Asn Tyr Lys His Lys Pro Ile Pro Asn Asn Asn Val Asn Leu Leu Ser
           355                 360                 365
   Tyr Phe Thr Asp Glu Leu Gln Asn Ile Gln Leu Ala Leu Tyr Thr Ala
       370                 375                 380
   Pro Pro Lys His Lys Ser Asp Thr Arg Asp Ser Tyr Thr Tyr Pro Tyr
   385                 390                 395                 400
   Gly Phe Ile Leu Asn Tyr Gln Asn Ser Lys Tyr Lys Tyr Gly Asp Asn
                   405                 410                 415
   Glu Pro Val Ile Ser Asn Thr Ile Ser Ala Pro Ile Gln Gln Ile Asn
               420                 425                 430
   Ala Ala Thr Gln Tyr Thr Gln Tyr Ile Asp Gly Glu Ser Ile Asn Gly
           435                 440                 445
   Ile Gly Ala Tyr Leu Pro Gly Tyr Cys Ser Thr Asp Cys Ser Glu Ile
       450                 455                 460
   Thr Pro Pro Phe Ala Cys Thr Ser Asn Asp Lys Asn Lys Ser Tyr Gly
   465                 470                 475                 480
   Ala Ser Cys Asn Ser Val Tyr Ser Ser Gln Lys Met Asn Ala Leu Tyr
                   485                 490                 495
   Pro Phe Thr Gln Thr Asn Val Pro Gly Asn Gln Gly Lys Leu Gly Val
               500                 505                 510
   Leu Ala Ser Tyr Val Pro Tyr Asp Leu Asn Pro Lys Asn Ile Phe Gly
           515                 520                 525
   Glu Val Asp Pro Asp Thr Asn Asn Ile Ile Leu Lys Gly Ile Pro Ala
       530                 535                 540
   Glu Lys Gly Tyr Phe Ser Asn Tyr Thr Arg Pro Thr Val Val Lys Glu
   545                 550                 555                 560
   Trp Ile Asn Gly Ala Asn Ala Val Ser Leu Tyr Ser Gly Asn Thr Leu
                   565                 570                 575
   Phe Ile Val Ala Thr Asn Ile Thr Ala Thr Gln Tyr Lys Ile Arg Ile
               580                 585                 590
   Arg Tyr Ala Asn Pro Asn Ser Asp Thr Glu Ile Ser Val Gln Ile Thr
           595                 600                 605
   Gln Asn Asn Ser Leu Leu His Ser Asp Thr Ile Thr Phe His Ser Thr
       610                 615                 620
   Thr Asp Ser Asn Met Asn Asn Asn Leu Ser Gln Asn Val Tyr Val Thr
   625                 630                 635                 640
   Gly Glu Asn Gly Asn Tyr Thr Leu Leu Asp Leu Tyr Asp Thr Thr Asn
                   645                 650                 655
   Val Leu Ser Thr Gly Asp Ile Thr Leu Gln Ile Thr Gly Gly Ser Gln
               660                 665                 670
   Glu Ile Phe Ile Asp Arg Ile Glu Phe Ile Pro Thr Ala Pro Ala Pro
           675                 680                 685
   Ala Pro Thr Asn Asp Asn Asn Asn Pro Pro Phe His Gly Cys Leu Ile
       690                 695                 700
   Ala Gly Glu Gln Gln Leu Cys Ser Gly Pro Pro Lys Phe Glu Gln Leu
   705                 710                 715                 720
   Ser Asp Leu Glu Lys Ile Thr Thr Gln Val Tyr Met Leu Phe Lys Ser
                   725                 730                 735
   Ser Ser Tyr Glu Glu Leu Asp Pro Lys Val Ser Ser Tyr Gln Ile Asn
               740                 745                 750
   Gln Val Ala Leu Lys Val Met Ser Leu Ser Asp Glu Met Phe Cys Glu
           755                 760                 765
   Glu Lys Arg Leu Leu Arg Lys Leu Val Asn Lys Ala Lys Gln Leu Val
       770                 775                 780
   Glu Ala Arg Asn Leu Leu Val Gly Gly Ser Phe Asp Thr Leu Gln Asn
   785                 790                 795                 800
   Trp Leu Leu Gly Thr Asn Ala Thr Ile Asn Tyr Asp Ser Phe Leu Phe
                   805                 810                 815
   Asn Gly Asn Tyr Leu Phe Leu Gln Pro Ala Ser Gly Phe Phe Ser Ser
               820                 825                 830
   Tyr Ala Tyr Gln Lys Ile Asn Glu Ser Lys Leu Lys Ser Tyr Thr Arg
           835                 840                 845
   Tyr Lys Val Ser Gly Phe Ile Gly Gln Ser Asn Gln Val Glu Leu Ile
       850                 855                 860
   Ile Ser Arg Tyr Gly Lys Glu Ile Asn Lys Ile Leu Asn Ile Ser Tyr
   865                 870                 875                 880
   Ala Gly Pro Leu Pro Ile Thr Ser Asn Thr Ser Thr Thr Cys Cys Ala
                   885                 890                 895
   Pro Asn Ile Gly Gln Cys Asn Glu Glu Gln Ser Asn Ser His Phe Phe
   900 905 910
   Ser Tyr Ser Ile Asp Val Gly Glu Leu Tyr Pro Glu Leu Asn Pro Gly
   915 920 925
   Ile Glu Phe Gly Leu Arg Ile Val Glu Pro Asn Ser Tyr Met Thr Ile
   930 935 940
   Ser Asn Leu Glu Ile Ile Glu Glu Arg Ser Leu Thr Glu Met Glu Ile
   945 950 955 960
   Gln Thr Ile Lys Arg Lys Asp Gln Lys Trp Lys Lys Glu Ile Leu Gln
   965 970 975
   Glu Cys Ala Asn Ile Asn Glu Leu Leu Gln Pro Ile Ile Asp Lys Val
   980 985 990
   Asp Ser Leu Phe Lys Asp Ala Asp Trp Tyr Gly Gln Ile Leu Pro His
   995 1000 1005
   Ile Thr Tyr Gln Asn Leu Lys Asn Ile Val Leu Pro Glu Leu Pro
   1010 1015 1020
   Lys Leu Arg His Trp Phe Ile Asn Asp Leu Ala Gly Glu Tyr Tyr
   1025 1030 1035
   Glu Ile Glu Gln Lys Ile Gln Gln Ala Leu Lys His Ala Phe Arg
   1040 1045 1050
   Gln Leu Asp Glu Arg Asn Leu Ile His Asn Gly His Phe Thr Ala
   1055 1060 1065
   Asn Leu Ile Asp Trp Gln Thr Glu Gly Asn Ala Gln Met Lys Ile
   1070 1075 1080
   Leu Glu Asn Gly Ala Leu Ala Ala Gln Leu Leu Ser Trp Asp Ser
   1085 1090 1095
   Ser Ile Ser Gln Ser Leu Asn Ile Leu Asp Phe Asp Glu Asp Lys
   1100 1105 1110
   Ala Tyr Lys Leu Arg Val Tyr Ala Gln Gly Ser Gly Thr Ile Gln
   1115 1120 1125
   Phe Lys Asn Cys Glu Asp Glu Thr Ile Gln Phe Asn Thr Asn Ser
   1130 1135 1140
   Phe Thr Tyr Lys Glu Lys Ile Phe Tyr Phe Asp Thr Pro Ser Ile
   1145 1150 1155
   Asn Leu Gln Ile Gln Ser Glu Gly Ser Asn Phe Val Ile Ser Ser
   1160 1165 1170
   Ile Glu Leu Ile Glu Leu Ser Ala Asp Glu
   1175 1180
<210> 3
   <211> 693
   <212> PRT
   <213> Bacillus thuringiensis
<400> 3
   Met Asp Cys Asn Leu Arg Ser Gln Gln Asn Ile Pro Tyr Asn Val Leu
   1 5 10 15
   Ala Thr Gln Ala Ser Asn Leu Ser Gln Phe Thr Asp Ile Ala Glu Gly
   20 25 30
   Val Lys Lys Ala Trp Ala Glu Phe Gln Lys Thr Gly Ser Phe Ser Leu
   35 40 45
   Glu Ala Leu Lys Gln Gly Phe Asn Ala Ala Gln Gly Gly Thr Phe Asn
   50 55 60
   Tyr Leu Ala Leu Leu Gln Ser Gly Ile Ser Leu Ala Gly Ser Phe Val
   65 70 75 80
   Pro Gly Gly Ser Phe Val Ala Pro Ile Val Asn Met Val Ile Gly Trp
   85 90 95
   Leu Trp Pro Asn Lys Asn Lys Thr Ala Asp Thr Glu Asn Leu Ile Lys
   100 105 110
   Leu Ile Asp Glu Glu Ile Gln Lys Gln Leu Asn Lys Ala Leu Leu Glu
   115 120 125
   Gln Asp Lys Asn Asn Trp Thr Ser Phe Leu Glu Ser Ile Phe Asp Val
   130 135 140
   Ser Asn Thr Val Asn Asn Ala Ile Ile Asp Ala Gln Trp Ser Gly Thr
   145 150 155 160
   Val Asp Asp Thr Asn Arg Gln Leu Lys Thr Pro Thr Thr Ser Asp Tyr
   165 170 175
   Lys Asn Val Val Glu Lys Phe Asp Ser Ala Asp Thr Ala Ile Ile Thr
   180 185 190
   Asn Glu Asn Gln Ile Met Asn Gly Asn Phe Asp Val Ala Ala Ser Ser
           195                 200                 205
   Tyr Phe Val Ile Gly Ala Thr Leu Arg Leu Ala Leu Phe Gln Ser Tyr
       210                 215                 220
   Ile Lys Phe Cys Asn His Trp Ile Asp Thr Val Gly Phe Asp Ser Asp
   225                 230                 235                 240
   Asn Tyr Asn Thr Gln Lys Ala Asn Leu Ala Arg Thr Lys Gln Thr Met
                   245                 250                 255
   Arg Thr Thr Ile Asn Asp Tyr Thr Gln Lys Ile Met Lys Val Phe Lys
               260                 265                 270
   Asn Ser Asp Asn Met Pro Thr Met Gly Thr Asn Lys Phe Ser Val Asp
           275                 280                 285
   Ala Tyr Asn Ala Tyr Ile Lys Gly Ile Thr Leu Asn Val Leu Asp Ile
       290                 295                 300
   Val Ser Thr Trp Pro Ser Leu Tyr Pro Asn Asp Tyr Thr Ser Gln Thr
   305                 310                 315                 320
   Lys Leu Glu Gln Thr Arg Ile Ile Phe Ser Asn Met Ile Gly Gln Gln
                   325                 330                 335
   Glu Ala Ile Asp Gly Thr Val Thr Ile Tyr Asp Thr Phe Asp Ser Asp
               340                 345                 350
   Asn Tyr Lys His Lys Pro Ile Pro Asn Asn Asn Val Asn Leu Leu Ser
           355                 360                 365
   Tyr Phe Thr Asp Glu Leu Gln Asn Ile Gln Leu Ala Leu Tyr Thr Ala
       370                 375                 380
   Pro Pro Lys His Lys Ser Asp Thr Arg Asp Ser Tyr Thr Tyr Pro Tyr
   385                 390                 395                 400
   Gly Phe Ile Leu Asn Tyr Gln Asn Ser Lys Tyr Lys Tyr Gly Asp Asn
                   405                 410                 415
   Glu Pro Val Ile Ser Asn Thr Ile Ser Ala Pro Ile Gln Gln Ile Asn
               420                 425                 430
   Ala Ala Thr Gln Tyr Thr Gln Tyr Ile Asp Gly Glu Ser Ile Asn Gly
           435                 440                 445
   Ile Gly Ala Tyr Leu Pro Gly Tyr Cys Ser Thr Asp Cys Ser Glu Ile
   450 455 460
   Thr Pro Pro Phe Ala Cys Thr Ser Asn Asp Lys Asn Lys Ser Tyr Gly
   465 470 475 480
   Ala Ser Cys Asn Ser Val Tyr Ser Ser Gln Lys Met Asn Ala Leu Tyr
   485 490 495
   Pro Phe Thr Gln Thr Asn Val Pro Gly Asn Gln Gly Lys Leu Gly Val
   500 505 510
   Leu Ala Ser Tyr Val Pro Tyr Asp Leu Asn Pro Lys Asn Ile Phe Gly
   515 520 525
   Glu Val Asp Pro Asp Thr Asn Asn Ile Ile Leu Lys Gly Ile Pro Ala
   530 535 540
   Glu Lys Gly Tyr Phe Ser Asn Tyr Thr Arg Pro Thr Val Val Lys Glu
   545 550 555 560
   Trp Ile Asn Gly Ala Asn Ala Val Ser Leu Tyr Ser Gly Asn Thr Leu
   565 570 575
   Phe Ile Val Ala Thr Asn Ile Thr Ala Thr Gln Tyr Lys Ile Arg Ile
   580 585 590
   Arg Tyr Ala Asn Pro Asn Ser Asp Thr Glu Ile Ser Val Gln Ile Thr
   595 600 605
   Gln Asn Asn Ser Leu Leu His Ser Asp Thr Ile Thr Phe His Ser Thr
   610 615 620
   Thr Asp Ser Asn Met Asn Asn Asn Leu Ser Gln Asn Val Tyr Val Thr
   625 630 635 640
   Gly Glu Asn Gly Asn Tyr Thr Leu Leu Asp Leu Tyr Asp Thr Thr Asn
   645 650 655
   Val Leu Ser Thr Gly Asp Ile Thr Leu Gln Ile Thr Gly Gly Ser Gln
   660 665 670
   Glu Ile Phe Ile Asp Arg Ile Glu Phe Ile Pro Thr Ala Pro Ala Pro
   675 680 685
   Ala Pro Thr Asn Asp
   690
<210> 4
   <211> 4
   <212> PRT
   <213> Artificial sequence
<220>
   <223> endoplasmic reticulum targeting peptide
<400> 4
   Lys Asp Glu Leu
   1

## Description

### CROSS REFERENCE TO RELATED APPLICATION

This application claims the benefit of U.S. Provisional Application Serial No. 61/503,132, filed June 30, 2011.

### REFERENCE TO SEQUENCE LISTING SUBMITTED ELECTRONICALLY

This official copy of the sequence listing is submitted electronically via EFS-Web s an ASCII formatted sequence listing with a file named "2916693-094977-SEQLIST.txt," created on June 28, 2012, and having a size of 21.4 kilobytes and is filed concurrently with the specification. The sequence listing contained in this ASCII formatted document is part of the specification.

### FIELD OF THE INVENTION

This invention relates to the field of molecular biology. Provided are novel genes that encode nematocidal proteins. These proteins and the nucleic acid sequences that encode them are useful in preparing nematocidal formulations and in the production of transgenic pest-resistant plants.

### BACKGROUND OF THE INVENTION

*Bacillus thuringiensis* is a Gram-positive spore forming soil bacterium characterized by its ability to produce crystalline inclusions that are specifically toxic to certain orders and species of insects, but are harmless to plants and other non-targeted organisms. For this reason, compositions including *Bacillus thuringiensis* strains or their insecticidal proteins can be used as environmentally-acceptable insecticides to control agricultural insect pests or insect vectors for a variety of human or animal diseases.

Crystal (Cry) proteins (delta-endotoxins) from *Bacillus thuringiensis* have potent insecticidal activity against predominantly Lepidopteran, Hemipteran, Dipteran, and Coleopteran larvae. These proteins also have shown activity against *Hymenoptera, Homoptera*, *Phthiraptera*, *Mallophaga*, and *Acari* pest orders, as well as other invertebrate orders such as *Nemathelminthes*, *Platyhelminthes*, and *Sarcomastigorphora* (Feitelson (1993) The Bacillus Thuringiensis family tree. In *Advanced Engineered Pesticides,* Marcel Dekker, Inc., New York, N.Y.) These proteins were originally classified as CryI to CryV based primarily on their insecticidal activity. The major classes were *Lepidoptera-specific* (I), *Lepidoptera-* and *Diptera-specific* (II), *Coleoptera-*specific (III), *Diptera-specific* (IV), and nematode-specific (V) and (VI). The proteins were further classified into subfamilies; more highly related proteins within each family were assigned divisional letters such as *Cry1A, Cry1B, Cry1C,* etc. Even more closely related proteins within each division were given names such as *Cry1C1, Cry1C2,* etc.

A new nomenclature was recently described for the Cry genes based upon amino acid sequence homology rather than insect target specificity (Crickmore et al. (1998) Microbiol. Mol. Biol. Rev. 62:807-813). In the new classification, each toxin is assigned a unique name incorporating a primary rank (an Arabic number), a secondary rank (an uppercase letter), a tertiary rank (a lowercase letter), and a quaternary rank (another Arabic number). In the new classification, Roman numerals have been exchanged for Arabic numerals in the primary rank. Proteins with less than 45% sequence identity have different primary ranks, and the criteria for secondary and tertiary ranks are 78% and 95%, respectively.

The crystal protein does not exhibit insecticidal activity until it has been ingested and solubilized in the insect midgut. The ingested protoxin is hydrolyzed by proteases in the insect digestive tract to an active toxic molecule. (Höfte and Whiteley (1989) Microbiol. Rev. 53:242-255). This toxin binds to apical brush border receptors in the midgut of the target larvae and inserts into the apical membrane creating ion channels or pores, resulting in larval death.

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, supra). Domains II and III are involved in receptor recognition and binding, and are therefore considered determinants of toxin specificity.

Because of the devastation that insects can confer, and the improvement in yield by controlling insect pests, there is a continual need to discover new forms of pesticidal toxins.

### SUMMARY OF INVENTION

Compositions and methods for conferring nematocidal activity to bacteria, plants, plant cells, tissues and seeds are provided. Compositions include nucleic acid molecules encoding sequences for nematocidal polypeptides, vectors comprising those nucleic acid molecules, and host cells comprising the vectors. Compositions also include the nematocidal polypeptide sequences and antibodies to those polypeptides. The nucleotide sequences can be used in DNA constructs or expression cassettes for transformation and expression in organisms, including microorganisms and plants. The nucleotide or amino acid sequences may be synthetic sequences that have been designed for expression in an organism including, but not limited to, a microorganism or a plant. Compositions also comprise bacteria, plants, plant cells, tissues, and seeds comprising the nucleotide sequence of the invention.

In particular, isolated nucleic acid molecules are provided that encode a pesticidal protein. Accordingly, the present invention provides a recombinant nucleic acid molecule comprising a nucleotide sequence encoding an amino acid sequence having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3.

Additionally, amino acid sequences corresponding to the pesticidal protein are encompassed. Hence, the present invention also provides a recombinant polypeptide with nematocidal activity, selected from the group consisting of:
a) a polypeptide comprising the amino acid sequence of any of SEQ ID NO:2 or 3; and
b) a polypeptide comprising an amino acid sequence having at least 95% sequence identity to the amino acid sequence of any of SEQ ID NO:2 or 3. In particular, the present invention provides for an isolated or recombinant nucleic acid molecule comprising a nucleotide sequence encoding the amino acid sequence shown in SEQ ID NO:2 or 3 or a nucleotide sequence set forth in SEQ ID NO:1, as well as biologically-active variants and fragments thereof which encode a polypeptide with, or have, at least 95% sequence identity to the amino acid sequence of any of SEQ ID NO: 2 or 3. Nucleotide sequences that are complementary to a nucleotide sequence of the invention, or that hybridize to a sequence of the invention or a complement thereof are also disclosed. Further provided are vectors, host cells, plants, and seeds comprising the nucleotide sequences of the invention, or nucleotide sequences encoding the amino acid sequences of the invention, as well as biologically-active variants and fragments thereof which encode a polypeptide with, or have, at least 95% sequence identity to the amino acid sequence of any of SEQ ID NO: 2 or 3.

The present invention also provides a method for controlling a nematode pest population or for killing a nematode pest, comprising contacting said population or pest with a pesticidally-effective amount of a polypeptide of the invention. Methods and kits for detecting the nucleic acids and polypeptides of the invention in a sample are also disclosed.

The compositions and methods of the invention are useful for the production of organisms with enhanced nematode pest resistance or tolerance. These organisms and compositions comprising the organisms are desirable for agricultural purposes. The compositions of the invention are also useful for generating altered or improved proteins that have pesticidal activity, or for detecting the presence of pesticidal proteins or nucleic acids in products or organisms.

The present invention also provides a method for producing a polypeptide with nematocidal activity, comprising culturing the host cell of claim 4 under conditions in which the nucleic acid molecule encoding the polypeptide is expressed.

The present invention also provides a plant having stably incorporated into its genome a DNA construct comprising a nucleotide sequence that encodes a protein having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3, preferably wherein said plant is a plant cell.

The present invention further provides a method for protecting a plant from a pest, comprising expressing in a plant or cell thereof a nucleotide sequence that encodes a nematocidal polypeptide, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3.

The present invention also provides a method for increasing yield in a plant comprising growing in a field a plant of or a seed thereof having stably incorporated into its genome a DNA construct comprising a nucleotide sequence that encodes a protein having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3;
wherein said field is infested with a nematode against which said polypeptide has nematocidal activity.

### DETAILED DESCRIPTION

The present invention is drawn to compositions and methods for regulating pest resistance or tolerance in organisms, particularly plants or plant cells. By "resistance" is intended that the pest, which is a nematode, is killed upon ingestion or other contact with the polypeptides of the invention. By "tolerance" is intended an impairment or reduction in the movement, feeding, reproduction, or other functions of the pest. The methods involve transforming organisms with a nucleotide sequence encoding a nematocidal protein of the invention. In particular, the nucleotide sequences of the invention are useful for preparing plants and microorganisms that possess pesticidal activity against nematodes. Thus, transformed bacteria, plants, plant cells, plant tissues and seeds are provided. Compositions are pesticidal nucleic acids and proteins of *Bacillus* or other species. The sequences find use in the construction of expression vectors for subsequent transformation into organisms of interest, as probes for the isolation of other homologous (or partially homologous) genes, and for the generation of altered pesticidal proteins by methods known in the art, such as domain swapping or DNA shuffling, for example, with members of the Cry1, Cry2, and Cry9 families of endotoxins. The proteins find use in controlling or killing nematode pest populations and for producing compositions with pesticidal activity.

By "pesticidal toxin" or "pesticidal protein" is intended a toxin that has toxic activity against one or more pests, including, but not limited to, members of the *Nematoda* phylum, or a protein that has homology to such a protein. Pesticidal proteins have been isolated from organisms including, for example, *Bacillus sp., Clostridium bifermentans* and *Paenibacillus popilliae.* Pesticidal proteins include amino acid sequences deduced from the full-length nucleotide sequences disclosed herein, and amino acid sequences that are shorter than the full-length sequences, either due to the use of an alternate downstream start site, or due to processing that produces a shorter protein having pesticidal activity, provided that they have nematocidal activity and at least 95% amino acid identity to the amino acid sequence of SEQ ID NO: 2 or 3. Processing may occur in the organism the protein is expressed in, or in the pest after ingestion of the protein.

Pesticidal proteins encompass delta-endotoxins. Delta-endotoxins include proteins identified as *cryl* through *cry43, cytl* and *cyt2,* and Cyt-like toxin. There are currently over 250 known species of delta-endotoxins with a wide range of specificities and toxicities. For an expansive list see Crickmore et al. (1998), Microbiol. Mol. Biol. Rev. 62:807-813, and for regular updates see Crickmore et al. (2003) "Bacillus thuringiensis toxin nomenclature," at www.biols.susx.ac.uk/Home/Neil_Crickmore/Bt/index.

Thus, provided herein are novel isolated or recombinant nucleotide sequences that confer nematocidal activity. These nucleotide sequences encode polypeptides with homology to known delta-endotoxins or binary toxins. Also provided are the amino acid sequences of the nematocidal proteins. The protein resulting from translation of this gene allows cells to control or kill pests that ingest it.

### Isolated Nucleic Acid Molecules, and Variants and Fragments Thereof

One aspect of the invention pertains to isolated or recombinant nucleic acid molecules comprising nucleotide sequences encoding pesticidal proteins and polypeptides or biologically active portions thereof. In particular, the present invention provides a recombinant nucleic acid molecule comprising a nucleotide sequence encoding an amino acid sequence having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3. Also disclosed are nucleic acid molecules sufficient for use as hybridization probes to identify nucleic acid molecules encoding proteins with regions of sequence homology. Also disclosed herein are nucleotide sequences capable of hybridizing to the nucleotide sequences of the invention under stringent conditions as defined elsewhere herein. As used herein, the term "nucleic acid molecule" is intended to include DNA molecules (e.g., recombinant DNA, cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" or "recombinant" nucleic acid sequence (or DNA) is used herein to refer to a nucleic acid sequence (or DNA) that is no longer in its natural environment, for example in an *in vitro* or in a recombinant bacterial or plant host cell. In some embodiments, an isolated or recombinant nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various embodiments, the isolated delta-endotoxin encoding nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. In various embodiments, a delta-endotoxin protein that is substantially free of cellular material includes preparations of protein having less than about 30%, 20%, 10%, or 5% (by dry weight) of non-delta-endotoxin protein (also referred to herein as a "contaminating protein").

Nucleotide sequences encoding the proteins of the present invention include the sequence set forth in SEQ ID NO:1, and variants, fragments, and complements thereof provided that they have nematocidal activity and at least 95% amino acid identity to the amino acid sequence of SEQ ID NO: 2 or 3. By "complement" is intended a nucleotide sequence that is sufficiently complementary to a given nucleotide sequence such that it can hybridize to the given nucleotide sequence to thereby form a stable duplex. The corresponding amino acid sequences for the pesticidal proteins encoded by these nucleotide sequences are set forth in SEQ ID NO:2 or 3.

Nucleic acid molecules that are fragments of these nucleotide sequences encoding pesticidal proteins are also encompassed by the present invention provided that they have nematocidal activity and at least 95% amino acid identity to the amino acid sequence of SEQ ID NO: 2 or 3. By "fragment" is intended a portion of the nucleotide sequence encoding a nematocidal protein. A fragment of a nucleotide sequence encodes a biologically active portion of a pesticidal protein. Also disclosed is a fragment that can be used as a hybridization probe or PCR primer using methods disclosed below. Nucleic acid molecules that are fragments of a nucleotide sequence encoding a pesticidal protein comprise at least about 50, 100, 200, 300, 400, 500, 600, 700, 800, 900, 1000, 1100, 1200, 1300, 1350, 1400 contiguous nucleotides, or up to the number of nucleotides present in a full-length nucleotide sequence encoding a pesticidal protein disclosed herein, depending upon the intended use. By "contiguous" nucleotides is intended nucleotide residues that are immediately adjacent to one another. Fragments of the nucleotide sequences of the present invention will encode protein fragments that retain the biological activity of the pesticidal protein and, hence, retain pesticidal activity against nematodes. Thus, biologically-active fragments of the polypeptides disclosed herein are also encompassed. By "retains activity" is intended that the fragment will have at least about 30%, at least about 50%, at least about 70%, 80%, 90%, 95% or higher of the pesticidal activity of the pesticidal protein. The pesticidal activity is nematocidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477.

A fragment of a nucleotide sequence encoding a pesticidal protein that encodes a biologically active portion of a protein of the invention will encode at least about 15, 25, 30, 50, 75, 100, 125, 150, 175, 200, 250, 300, 350, 400, 450 contiguous amino acids, or up to the total number of amino acids present in a full-length pesticidal protein of the invention. In some embodiments, the fragment is a proteolytic cleavage fragment. For example, the proteolytic cleavage fragment may have an N-terminal or a C-terminal truncation of at least about 100 amino acids, about 120, about 130, about 140, about 150, or about 160 amino acids relative to SEQ ID NO:2 or 3. In some embodiments, the fragments encompassed herein result from the removal of the C-terminal crystallization domain, e.g., by proteolysis or by insertion of a stop codon in the coding sequence.

Preferred pesticidal proteins of the present invention are encoded by a nucleotide sequence sufficiently identical to the nucleotide sequence of SEQ ID NO:1, or the pesticidal proteins are sufficiently identical to the amino acid sequence set forth in SEQ ID NO:2 or 3. By "sufficiently identical" is intended an amino acid or nucleotide sequence that has at least 95%, 96%, 97%, 98%, 99% or greater sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. In another embodiment, the percent identity is calculated across the entirety of the reference sequence (i.e., the sequence disclosed herein as any of SEQ ID NO:1-3). The percent identity between two sequences can be determined using techniques similar to those described below, with or without allowing gaps. In calculating percent identity, typically exact matches are counted. A gap, i.e. a position in an alignment where a residue is present in one sequence but not in the other, is regarded as a position with non-identical residues.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain nucleotide sequences homologous to pesticidal-like nucleic acid molecules of the invention. BLAST protein searches can be performed with the BLASTX program, score = 50, wordlength = 3, to obtain amino acid sequences homologous to pesticidal protein molecules of the invention. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. See Altschul *et al.* (1997) *supra.* When utilizing BLAST, Gapped BLAST, and PSI-Blast programs, the default parameters of the respective programs (e.g., BLASTX and BLASTN) can be used. Alignment may also be performed manually by inspection.

Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GENEDOC™. GENEDOC™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG Wisconsin Genetics Software Package, Version 10 (available from Accelrys, Inc., 9685 Scranton Rd., San Diego, CA, USA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) J. Mol. Biol. 48(3):443-453, will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

The invention also encompasses variant nucleic acid molecules provided that they have nematocidal activity and at least 95% amino acid identity to the amino acid sequence of SEQ ID NO: 2 or 3. "Variants" of the pesticidal protein encoding nucleotide sequences include those sequences that encode the pesticidal proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the pesticidal proteins disclosed in the present invention as discussed below. Variant proteins encompassed by the present invention are biologically active, that is they continue to possess the desired biological activity of the native protein, that is, pesticidal activity. By "retains activity" is intended that the variant will have at least about 30%, at least about 50%, at least about 70%, or at least about 80% of the pesticidal activity of the native protein. Methods for measuring pesticidal activity are well known in the art. See, for example, Czapla and Lang (1990) J. Econ. Entomol. 83: 2480-2485; Andrews et al. (1988) Biochem. J. 252:199-206; Marrone et al. (1985) J. of Economic Entomology 78:290-293; and U.S. Patent No. 5,743,477.

The skilled artisan will further appreciate that changes can be introduced by mutation of the nucleotide sequences of the invention thereby leading to changes in the amino acid sequence of the encoded pesticidal proteins, without altering the biological activity of the proteins provided that they have nematocidal activity and at least 95% amino acid identity to the amino acid sequence of SEQ ID NO: 2 or 3. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, conservative amino acid substitutions may be made at one or more, predicted, nonessential amino acid residues. A "nonessential" amino acid residue is a residue that can be altered from the wild-type sequence of a pesticidal protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, supra). Domains II and III are involved in receptor recognition and binding, and are therefore considered determinants of toxin specificity.

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in an alignment of similar or related toxins to the sequences of the invention (e.g., residues that are identical in an alignment of homologous proteins). Examples of residues that are conserved but that may allow conservative amino acid substitutions and still retain activity include, for example, residues that have only conservative substitutions between all proteins contained in an alignment of similar or related toxins to the sequences of the invention (e.g., residues that have only conservative substitutions between all proteins contained in the alignment homologous proteins). However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for ability to confer pesticidal activity to identify mutants that retain activity. Following mutagenesis, the encoded protein can be expressed recombinantly, and the activity of the protein can be determined using standard assay techniques.

Using methods such as PCR, hybridization, and the like corresponding pesticidal sequences can be identified, such sequences having substantial identity to the sequences of the invention. See, for example, Sambrook and Russell (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the pesticidal nucleotide sequence can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known pesticidal protein-encoding nucleotide sequence disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, at least about 25, at least about 50, 75, 100, 125, 150, 175, or 200 consecutive nucleotides of nucleotide sequence encoding a pesticidal protein of the invention or a fragment or variant thereof. Methods for the preparation of probes for hybridization are generally known in the art and are disclosed in Sambrook and Russell, 2001.

For example, an entire pesticidal sequence disclosed herein, or one or more portions thereof, may be used as a probe capable of specifically hybridizing to corresponding pesticidal protein-like sequences and messenger RNAs. To achieve specific hybridization under a variety of conditions, such probes include sequences that are unique and are preferably at least about 10 nucleotides in length, or at least about 20 nucleotides in length. Such probes may be used to amplify corresponding pesticidal sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Thus, also disclosed are probes for hybridization, as well as nucleotide sequences capable of hybridization to all or a portion of a nucleotide sequence of the invention (e.g., at least about 300 nucleotides, at least about 400, at least about 500, 1000, 1200, 1500, 2000, 2500, 3000, 3500, or up to the full length of a nucleotide sequence disclosed herein). Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, New York).

### Isolated Proteins and Variants and Fragments Thereof

Pesticidal proteins are also encompassed within the present invention. By "pesticidal protein" is intended a protein having the amino acid sequence set forth in SEQ ID NO:2 or 3. Fragments, biologically active portions, and variants thereof are also provided, and may be used to practice the methods of the present invention provided that they have nematocidal activity and at least 95% amino acid identity to the amino acid sequence of SEQ ID NO: 2 or 3. An "isolated protein" or a "recombinant protein" is used to refer to a protein that is no longer in its natural environment, for example in vitro or in a recombinant bacterial or plant host cell.

"Fragments" or "biologically active portions" include polypeptide fragments comprising amino acid sequences sufficiently identical to the amino acid sequence set forth in SEQ ID NO:2 or 3, and that exhibit pesticidal activity. A biologically active portion of a pesticidal protein can be a polypeptide that is, for example, 10, 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, or more amino acids in length. Such biologically active portions can be prepared by recombinant techniques and evaluated for pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743477. As used here, a fragment comprises at least 8 contiguous amino acids of SEQ ID NO:2 or 3. The invention encompasses other fragments, however, such as any fragment in the protein greater than about 10, 20, 30, 50, 100, 150, 200, 250, 300, 350, 400, 450, 500, 550, 600, 650, 700, 750, 800, 850, 900, 950, 1000, 1050, 1100, 1150, 1200, 1250, 1300, 1350 or more amino acids in length.

By "variants" is intended proteins or polypeptides having an amino acid sequence that is at least 95%, 96%, 97%, 98% or 99% identical to the amino acid sequence of any of SEQ ID NO:2 or 3. Variants also include polypeptides encoded by a nucleic acid molecule that hybridizes to the nucleic acid molecule of SEQ ID NO:1, or a complement thereof, under stringent conditions provided that they have nematocidal activity and at least 95% amino acid identity to the amino acid sequence of SEQ ID NO: 2 or 3. Variants include polypeptides that differ in amino acid sequence due to mutagenesis. Variant proteins encompassed by the present invention are biologically active, that is they continue to possess the desired biological activity of the native protein, that is, retaining pesticidal activity against nematodes. In some embodiments, the variants have improved activity relative to the native protein. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477.

Bacterial genes, such as the *axmi* genes of this invention, quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. On rare occasions, translation in bacterial systems can initiate at a TTG codon, though in this event the TTG encodes a methionine. Furthermore, it is not often determined *a priori* which of these codons are used naturally in the bacterium. Thus, it is understood that use of one of the alternate methionine codons may also lead to generation of pesticidal proteins. These pesticidal proteins are encompassed in the present invention and may be used in the methods of the present invention. It will be understood that, when expressed in plants, it will be necessary to alter the alternate start codon to ATG for proper translation.

Antibodies to the polypeptides of the present invention, or to variants or fragments thereof, are also disclosed. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

Antibodies to the polypeptides of the present invention, or to variants or fragments thereof, are also disclosed. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

Thus, also disclosed are antibodies, single-chain antigen binding molecules, or other proteins that specifically bind to one or more of the protein or peptide molecules of the invention and their homologs, fusions or fragments. In a particularly preferred instance, the antibody specifically binds to a protein having the amino acid sequence set forth in SEQ ID NO:2 or 3, or a fragment thereof. In another instance, the antibody specifically binds to a fusion protein comprising an amino acid sequence selected from the amino acid sequence set forth in SEQ ID NO:2 or 3, or a fragment thereof.

Antibodies may be used to quantitatively or qualitatively detect the protein or peptide molecules of the invention, or to detect post translational modifications of the proteins. As used herein, an antibody or peptide is said to "specifically bind" to a protein or peptide molecule of the invention if such binding is not competitively inhibited by the presence of non-related molecules.

### Altered or Improved Variants

It is recognized that DNA sequences of a pesticidal protein may be altered by various methods, and that these alterations may result in DNA sequences encoding proteins with amino acid sequences different than that encoded by a pesticidal protein of the present invention provided that they have nematocidal activity and at least 95% amino acid identity to the amino acid sequence of SEQ ID NO: 2 or 3. This protein may be altered in various ways including amino acid substitutions, deletions, truncations, and insertions of one or more amino acids of SEQ ID NO:2 or 3, including up to about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 100, about 105, about 110, about 115, about 120, about 125, about 130, about 135, about 140, about 145, about 150, about 155, or more amino acid substitutions, deletions or insertions. Methods for such manipulations are generally known in the art. For example, amino acid sequence variants of a pesticidal protein can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a pesticidal protein to confer pesticidal activity may be improved by the use of such techniques upon the compositions of this invention. For example, one may express a pesticidal protein in host cells that exhibit high rates of base misincorporation during DNA replication, such as XL-1 Red (Stratagene, La Jolla, CA). After propagation in such strains, one can isolate the DNA (for example by preparing plasmid DNA, or by amplifying by PCR and cloning the resulting PCR fragment into a vector), culture the pesticidal protein mutations in a non-mutagenic strain, and identify mutated genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. This can include insertions, deletions, or alterations introduced by modern molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different pesticidal protein coding regions can be used to create a new pesticidal protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between a pesticidal gene of the invention and other known pesticidal genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

Domain swapping or shuffling is another mechanism for generating altered pesticidal proteins. Domains may be swapped between pesticidal proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepfet al. (1990) J. Biol. Chem. 265:20923-20930; Rang et al. 91999) Appl. Environ. Microbiol. 65:2918-2925).

### Vectors

A pesticidal sequence of the invention may be provided in an expression cassette for expression in a plant of interest. By "plant expression cassette" is intended a DNA construct that is capable of resulting in the expression of a protein from an open reading frame in a plant cell. Typically these contain a promoter and a coding sequence. Often, such constructs will also contain a 3' untranslated region. Such constructs may contain a "signal sequence" or "leader sequence" to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this typically involves secretion into the Golgi apparatus, with some resulting glycosylation. Insecticidal toxins of bacteria are often synthesized as protoxins, which are protolytically activated in the gut of the target pest (Chang (1987) Methods Enzymol. 153:507-516). In some embodiments of the present invention, the signal sequence is located in the native sequence, or may be derived from a sequence of the invention. By "leader sequence" is intended any sequence that when translated, results in an amino acid sequence sufficient to trigger co-translational transport of the peptide chain to a subcellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell. The cassette will include 5' and/or 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

In various embodiments, the nucleotide sequence of the invention is operably linked to a promoter, e.g., a plant promoter. "Promoter" refers to a nucleic acid sequence that functions to direct transcription of a downstream coding sequence. The promoter together with other transcriptional and translational regulatory nucleic acid sequences (also termed "control sequences") are necessary for the expression of a DNA sequence of interest.

Such an expression cassette is provided with a plurality of restriction sites for insertion of the pesticidal sequence to be under the transcriptional regulation of the regulatory regions.

The expression cassette will include in the 5'-3' direction of transcription, a transcriptional and translational initiation region (i.e., a promoter), a DNA sequence of the invention, and a translational and transcriptional termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed host cell. That is, the genes can be synthesized using host cell-preferred codons for improved expression, or may be synthesized using codons at a host-preferred codon usage frequency. Generally, the GC content of the gene will be increased. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, U.S. Patent Publication No. 20090137409, and Murray et al. (1989) Nucleic Acids Res. 17:477-498.

In one embodiment, the pesticidal protein is targeted to the chloroplast for expression. In this manner, where the pesticidal protein is not directly inserted into the chloroplast, the expression cassette will additionally contain a nucleic acid encoding a transit peptide to direct the pesticidal protein to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The pesticidal gene to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

### Plant Transformation

Methods of the invention involve introducing a nucleotide construct into a plant. By "introducing" is intended to present to the plant the nucleotide construct in such a manner that the construct gains access to the interior of a cell of the plant. The methods of the invention do not require that a particular method for introducing a nucleotide construct to a plant is used, only that the nucleotide construct gains access to the interior of at least one cell of the plant. Methods for introducing nucleotide constructs into plants are known in the art including, but not limited to, stable transformation methods, transient transformation methods, and virus-mediated methods.

By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen).

"Transgenic plants" or "transformed plants" or "stably transformed" plants or cells or tissues refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

The transgenic plants of the invention express one or more of the novel toxin sequences disclosed herein. In various embodiments, the transgenic plant further comprises one or more additional genes for insect resistance (e.g., Cry1, such as members of the Cry1A, Cry1B, Cry1C, Cry1D, Cry1E, and Cry1F families; Cry2, such as members of the Cry2A family; Cry9, such as members of the Cry9A, Cry9B, Cry9C, Cry9D, Cry9E, and Cry9F families; etc.). It will be understood by one of skill in the art that the transgenic plant may comprise any gene imparting an agronomic trait of interest.

Transformation of plant cells can be accomplished by one of several techniques known in the art. The pesticidal gene of the invention may be modified to obtain or enhance expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this "plant expression cassette" will be inserted into a "plant transformation vector". This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors." Binary vectors as well as vectors with helper plasmids are most often used for *Agrobacterium-mediated* transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the pesticidal gene are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science 5:446-451). Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Transformation protocols as well as protocols for introducing nucleotide sequences into plants may vary depending on the type of plant or plant cell, i.e., monocot or dicot, targeted for transformation. Generation of transgenic plants may be performed by one of several methods, including, but not limited to, microinjection, electroporation, direct gene transfer, introduction of heterologous DNA by *Agrobacterium* into plant cells (*Agrobacterium*-mediated transformation), bombardment of plant cells with heterologous foreign DNA adhered to particles, ballistic particle acceleration, aerosol beam transformation (U.S. Published Application No. 20010026941; U.S. Patent No. 4,945,050; International Publication No. WO 91/00915; U.S. Published Application No. 2002015066), Lec1 transformation, and various other non-particle direct-mediated methods to transfer DNA.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Molecular and biochemical methods can then be used to confirm the presence of the integrated heterologous gene of interest into the genome of the transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR analysis is a rapid method to screen transformed cells, tissue or shoots for the presence of incorporated gene at the earlier stage before transplanting into the soil (Sambrook and Russell (2001) Molecular Cloning: A Laboratory Manual. Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra*). In general, total DNA is extracted from the transformant, digested with appropriate restriction enzymes, fractionated in an agarose gel and transferred to a nitrocellulose or nylon membrane. The membrane or "blot" is then probed with, for example, radiolabeled ³²P target DNA fragment to confirm the integration of introduced gene into the plant genome according to standard techniques (Sambrook and Russell, 2001, *supra).*

In Northern blot analysis, RNA is isolated from specific tissues of transformant, fractionated in a formaldehyde agarose gel, and blotted onto a nylon filter according to standard procedures that are routinely used in the art (Sambrook and Russell, 2001, *supra*). Expression of RNA encoded by the pesticidal gene is then tested by hybridizing the filter to a radioactive probe derived from a pesticidal gene, by methods known in the art (Sambrook and Russell, 2001, *supra).*

Western blot, biochemical assays and the like may be carried out on the transgenic plants to confirm the presence of protein encoded by the pesticidal gene by standard procedures (Sambrook and Russell, 2001, *supra)* using antibodies that bind to one or more epitopes present on the pesticidal protein.

### Pesticidal Activity in Plants

In another aspect of the invention, one may generate transgenic plants expressing a pesticidal protein that has nematocidal activity. Methods described above by way of example may be utilized to generate transgenic plants, but the manner in which the transgenic plant cells are generated is not critical to this invention. Methods known or described in the art such as *Agrobacterium-mediated* transformation, biolistic transformation, and non-particle-mediated methods may be used at the discretion of the experimenter. Plants expressing a nematocidal protein may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene). Additionally, the genes disclosed herein are useful as markers to assess transformation of bacterial or plant cells. Methods for detecting the presence of a transgene in a plant, plant organ (e.g., leaves, stems, roots, etc.), seed, plant cell, propagule, embryo or progeny of the same are well known in the art. In one embodiment, the presence of the transgene is detected by testing for pesticidal activity.

Fertile plants expressing a nematocidal protein may be tested for nematocidal activity, and the plants showing optimal activity selected for further breeding. Methods are available in the art to assay for nematode pest activity. Generally, the protein is mixed and used in feeding assays. See, for example Marrone et al. (1985) J. of Economic Entomology 78:290-293.

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Preferably, plants of the present invention are crop plants (for example, maize, sorghum, wheat, sunflower, tomato, crucifers, peppers, potato, cotton, rice, soybean, sugarbeet, sugarcane, tobacco, barley, oilseed rape., etc.).

### Use in Pesticidal Control

General methods for employing strains comprising a nucleotide sequence of the present invention, or a variant thereof, in nematode pest control or in engineering other organisms as nematocidal agents are known in the art. See, for example U.S. Patent No. 5,039,523 and EP 0480762A2.

The *Bacillus* strains containing a nucleotide sequence of the present invention, or a variant thereof, or the microorganisms that have been genetically altered to contain a nematocidal gene of the invention and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticide is produced by introducing a nematocidal gene into a cellular host. Expression of the pesticidal gene results, directly or indirectly, in the intracellular production and maintenance of the pesticide. In one aspect of this invention, these cells are then treated under conditions that prolong the activity of the toxin produced in the cell when the cell is applied to the environment of the target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing a gene of this invention such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, molluscicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Methods of applying an active ingredient of the present invention or an agrochemical composition of the present invention that contains at least one of the pesticidal proteins produced by the bacterial strains of the present invention include leaf application, seed coating and soil application. The number of applications and the rate of application depend on the intensity of infestation by the corresponding pest.

The composition may be formulated as a powder, dust, pellet, granule, spray, emulsion, colloid, solution, or such like, and may be prepared by such conventional means as desiccation, lyophilization, homogenation, extraction, filtration, centrifugation, sedimentation, or concentration of a culture of cells comprising the polypeptide. In all such compositions that contain at least one such nematocidal polypeptide, the polypeptide may be present in a concentration of from about 1% to about 99% by weight.

Nematode pests may be killed or reduced in numbers in a given area by the methods of the invention, or may be prophylactically applied to an environmental area to prevent infestation by a susceptible pest. Preferably the pest ingests, or is contacted with, a pesticidally-effective amount of the polypeptide. By "pesticidally-effective amount" is intended an amount of the pesticide that is able to bring about death to at least one nematode pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating either the bacterial cell, the crystal and/or the spore suspension, or the isolated protein component with the desired agriculturally-acceptable carrier. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No. 6,468,523.

"Pest" includes but is not limited to, insects, fungi, bacteria, nematodes, mites, ticks, and the like. The polypeptides of the invention have nematocidal activity.

Insect pests include insects selected from the orders *Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera,* etc., particularly *Coleoptera, Lepidoptera,* and *Diptera.*

The order *Coleoptera* includes the suborders *Adephaga* and *Polyphaga.* Suborder *Adephaga* includes the superfamilies *Caraboidea* and *Gyrinoidea,* while suborder *Polyphaga* includes the superfamilies *Hydrophiloidea, Staphylinoidea*, *Cantharoidea*, *Cleroidea*, *Elateroidea*, *Dascilloidea*, *Dryopoidea*, *Byrrhoidea*, *Cucujoidea*, *Meloidea*, *Mordelloidea*, *Tenebrionoidea*, *Bostrichoidea*, *Scarabaeoidea*, *Cerambycoidea, Chrysomeloidea,* and *Curculionoidea*. Superfamily *Caraboidea* includes the families *Cicindelidae, Carabidae,* and *Dytiscidae*. Superfamily *Gyrinoidea* includes the family *Gyrinidae.* Superfamily *Hydrophiloidea* includes the family *Hydrophilidae*. Superfamily *Staphylinoidea* includes the families *Silphidae* and *Staphylinidae*. Superfamily *Cantharoidea* includes the families *Cantharidae* and *Lampyridae*. Superfamily *Cleroidea* includes the families *Cleridae* and *Dermestidae*. Superfamily *Elateroidea* includes the families *Elateridae* and *Buprestidae*. Superfamily *Cucujoidea* includes the family *Coccinellidae*. Superfamily *Meloidea* includes the family *Meloidae*. Superfamily *Tenebrionoidea* includes the family *Tenebrionidae*. Superfamily *Scarabaeoidea* includes the families *Passalidae* and *Scarabaeidae*. Superfamily *Cerambycoidea* includes the family *Cerambycidae*. Superfamily *Chrysomeloidea* includes the family *Chrysomelidae*. Superfamily *Curculionoidea* includes the families *Curculionidae* and *Scolytidae*.

The order *Diptera* includes the Suborders *Nematocera*, *Brachycera*, and *Cyclorrhapha*. Suborder *Nematocera* includes the families *Tipulidae*, *Psychodidae*, *Culicidae*, *Ceratopogonidae*, *Chironomidae*, *Simuliidae*, *Bibionidae*, and *Cecidomyiidae*. Suborder *Brachycera* includes the families *Stratiomyidae*, *Tabanidae*, *Therevidae*, *Asilidae*, *Mydidae*, *Bombyliidae*, and *Dolichopodidae*. Suborder *Cyclorrhapha* includes the Divisions *Aschiza* and *Aschiza.* Division *Aschiza* includes the families *Phoridae*, *Syrphidae*, and *Conopidae.* Division *Aschiza* includes the Sections *Acalyptratae* and *Calyptratae.* Section *Acalyptratae* includes the families *Otitidae*, *Tephritidae*, *Agromyzidae*, and *Drosophilidae*. Section *Calyptratae* includes the families *Hippoboscidae*, Oestridae, *Tachinidae*, *Anthomyiidae*, *Muscidae*, *Calliphoridae*, and *Sarcophagidae*.

The order *Lepidoptera* includes the families *Papilionidae*, *Pieridae*, *Lycaenidae*, *Nymphalidae*, *Danaidae*, *Satyridae*, *Hesperiidae*, *Sphingidae*, *Saturniidae*, *Geometridae*, *Arctiidae*, *Noctuidae*, *Lymantriidae*, *Sesiidae*, and *Tineidae*.

Insect pests of the invention for the major crops include: Maize: *Ostrinia nubilalis*, European corn borer; *Agrotis ipsilon*, black cutworm; *Helicoverpa zea,* corn earworm; *Spodoptera frugiperda*, fall armyworm; *Diatraea grandiosella*, southwestern corn borer; *Elasmopalpus lignosellus*, lesser cornstalk borer; *Diatraea saccharalis*, surgarcane borer; *Diabrotica virgifera*, western corn rootworm; *Diabrotica longicornis barberi*, northern corn rootworm; *Diabrotica undecimpunctata howardi*, southern corn rootworm; *Melanotus* spp., wireworms; *Cyclocephala borealis*, northern masked chafer (white grub); *Cyclocephala immaculata*, southern masked chafer (white grub); *Popillia japonica*, Japanese beetle; *Chaetocnema pulicaria*, corn flea beetle; *Sphenophorus maidis*, maize billbug; *Rhopalosiphum maidis*, corn leaf aphid; *Anuraphis maidiradicis*, corn root aphid; *Blissus leucopterus leucopterus*, chinch bug; *Melanoplus femurrubrum*, redlegged grasshopper; *Melanoplus sanguinipes*, migratory grasshopper; *Hylemya platura*, seedcorn maggot; *Agromyza parvicornis*, corn blot leafminer; *Anaphothrips obscrurus*, grass thrips; *Solenopsis milesta*, thief ant; *Tetranychus urticae*, twospotted spider mite; Sorghum: *Chilo partellus,* sorghum borer; *Spodoptera frugiperda*, fall armyworm; *Helicoverpa zea,* corn earworm; *Elasmopalpus lignosellus,* lesser cornstalk borer; *Feltia subterranea*, granulate cutworm; *Phyllophaga crinita*, white grub; *Eleodes*, *Conoderus*, and *Aeolus* spp., wireworms; *Oulema melanopus*, cereal leaf beetle; *Chaetocnema pulicaria*, corn flea beetle; *Sphenophorus maidis*, maize billbug; *Rhopalosiphum maidis*; corn leaf aphid; *Sipha flava*, yellow sugarcane aphid; *Blissus leucopterus leucopterus*, chinch bug; *Contarinia sorghicola*, sorghum midge; *Tetranychus cinnabarinus*, carmine spider mite; *Tetranychus urticae*, twospotted spider mite; Wheat: *Pseudaletia unipunctata*, army worm; *Spodoptera frugiperda*, fall armyworm; *Elasmopalpus lignosellus*, lesser cornstalk borer; *Agrotis orthogonia*, western cutworm; *Elasmopalpus lignosellus*, lesser cornstalk borer; *Oulema melanopus,* cereal leaf beetle; *Hypera punctata*, clover leaf weevil; *Diabrotica undecimpunctata howardi*, southern corn rootworm; Russian wheat aphid; *Schizaphis graminum*, greenbug; *Macrosiphum avenae*, English grain aphid; *Melanoplus femurrubrum*, redlegged grasshopper; *Melanoplus differentialis*, differential grasshopper; *Melanoplus sanguinipes*, migratory grasshopper; *Mayetiola destructor,* Hessian fly; *Sitodiplosis mosellana*, wheat midge; *Meromyza americana*, wheat stem maggot; *Hylemya coarctata*, wheat bulb fly; *Frankliniella fusca*, tobacco thrips; *Cephus cinctus*, wheat stem sawfly; *Aceria tulipae*, wheat curl mite; Sunflower: *Suleima helianthana*, sunflower bud moth; *Homoeosoma electellum*, sunflower moth; *zygogramma exclamationis*, sunflower beetle; *Bothyrus gibbosus*, carrot beetle; *Neolasioptera murtfeldtiana*, sunflower seed midge; Cotton: *Heliothis virescens*, cotton budworm; *Helicoverpa zea,* cotton bollworm; *Spodoptera exigua*, beet armyworm; *Pectinophora gossypiella*, pink bollworm; *Anthonomus grandis*, boll weevil; *Aphis gossypii*, cotton aphid; *Pseudatomoscelis seriatus*, cotton fleahopper; *Trialeurodes abutilonea*, bandedwinged whitefly; *Lygus lineolaris*, tarnished plant bug; *Melanoplus femurrubrum*, redlegged grasshopper; *Melanoplus differentialis*, differential grasshopper; *Thrips tabaci,* onion thrips; *Franklinkiella fusca*, tobacco thrips; *Tetranychus cinnabarinus*, carmine spider mite; *Tetranychus urticae*, twospotted spider mite; Rice: *Diatraea saccharalis*, sugarcane borer; *Spodoptera frugiperda*, fall armyworm; *Helicoverpa zea,* corn earworm; *Colaspis brunnea*, grape colaspis; *Lissorhoptrus oryzophilus*, rice water weevil; *Sitophilus oryzae*, rice weevil; *Nephotettix nigropictus*, rice leafhopper; *Blissus leucopterus leucopterus*, chinch bug; *Acrosternum hilare*, green stink bug; Soybean: *Pseudoplusia includens*, soybean looper; *Anticarsia gemmatalis*, velvetbean caterpillar; *Plathypena scabra*, green cloverworm; *Ostrinia nubilalis*, European corn borer; *Agrotis ipsilon*, black cutworm; *Spodoptera exigua*, beet armyworm; *Heliothis virescens*, cotton budworm; *Helicoverpa zea,* cotton bollworm; *Epilachna varivestis*, Mexican bean beetle; *Myzus persicae*, green peach aphid; *Empoascafabae*, potato leafhopper; *Acrosternum hilare*, green stink bug; *Melanoplus femurrubrum*, redlegged grasshopper; *Melanoplus differ entialis*, differential grasshopper; *Hylemya platura*, seedcorn maggot; *Sericothrips variabilis*, soybean thrips; *Thrips tabaci,* onion thrips; *Tetranychus turkestani*, strawberry spider mite; *Tetranychus urticae*, twospotted spider mite; Barley: *Ostrinia nubilalis,* European corn borer; *Agrotis ipsilon*, black cutworm; *Schizaphis graminum*, greenbug; *Blissus leucopterus leucopterus,* chinch bug; *Acrosternum hilare*, green stink bug; *Euschistus servus*, brown stink bug; *Delia platura*, seedcorn maggot; *Mayetiola destructor,* Hessian fly; *Petrobia latens*, brown wheat mite; Oil Seed Rape: *Brevicoryne brassicae*, cabbage aphid; *Phyllotreta cruciferae*, Flea beetle; *Mamestra configurata*, Bertha armyworm; *Plutella xylostella*, Diamond-back moth; *Delia* ssp., Root maggots.

Nematodes include parasitic nematodes such as root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

### Methods for Increasing Plant Yield

Methods for increasing plant yield are provided. The methods comprise providing a plant or plant cell expressing a polynucleotide encoding the nematocidal polypeptide sequence disclosed herein and growing the plant or a seed thereof in a field infested with (or susceptible to infestation by) a pest against which said polypeptide has nematocidal activity. In some embodiments, the polypeptide has pesticidal activity against a nematode pest, and said field is infested with a nematode pest. As defined herein, the "yield" of the plant refers to the quality and/or quantity of biomass produced by the plant. By "biomass" is intended any measured plant product. An increase in biomass production is any improvement in the yield of the measured plant product. Increasing plant yield has several commercial applications. For example, increasing plant leaf biomass may increase the yield of leafy vegetables for human or animal consumption. Additionally, increasing leaf biomass can be used to increase production of plant-derived pharmaceutical or industrial products. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase in yield compared to a plant not expressing the pesticidal sequence. In specific methods, plant yield is increased as a result of improved nematode pest resistance of a plant expressing a nematocidal protein disclosed herein. Expression of the nematocidal protein results in a reduced ability of a nematode pest to infest or feed.

The plants can also be treated with one or more chemical compositions, including one or more herbicide, insecticides, or fungicides. Exemplary chemical compositions include: Fruits/Vegetables Herbicides: Atrazine, Bromacil, Diuron, Glyphosate, Linuron, Metribuzin, Simazine, Trifluralin, Fluazifop, Glufosinate, Halosulfuron Gowan, Paraquat, Propyzamide, Sethoxydim, Butafenacil, Halosulfuron, Indaziflam; Fruits/Vegetables Insecticides: Aldicarb , Bacillus thuriengiensis, Carbaryl, Carbofuran, Chlorpyrifos, Cypermethrin, Deltamethrin, Abamectin, Cyfluthrin/beta-cyfluthrin, Esfenvalerate, Lambda-cyhalothrin, Acequinocyl, Bifenazate, Methoxyfenozide, Novaluron, Chromafenozide, Thiacloprid, Dinotefuran, Fluacrypyrim, Spirodiclofen, Gamma-cyhalothrin, Spiromesifen, Spinosad, Rynaxypyr, Cyazypyr, Triflumuron,Spirotetramat, Imidacloprid, Flubendiamide, Thiodicarb, Metaflumizone, Sulfoxaflor, Cyflumetofen, Cyanopyrafen, Clothianidin, Thiamethoxam, Spinotoram, Thiodicarb, Flonicamid, Methiocarb, Emamectin-benzoate, Indoxacarb, Fenamiphos, Pyriproxifen, Fenbutatin-oxid; Fruits/Vegetables Fungicides: Ametoctradin, Azoxystrobin, Benthiavalicarb, Boscalid, Captan, Carbendazim, Chlorothalonil, Copper, Cyazofamid, Cyflufenamid, Cymoxanil, Cyproconazole, Cyprodinil, Difenoconazole, Dimetomorph, Dithianon, Fenamidone, Fenhexamid, Fluazinam, Fludioxonil, Fluopicolide, Fluopyram, Fluoxastrobin, Fluxapyroxad, Folpet, Fosetyl, Iprodione, Iprovalicarb, Isopyrazam, Kresoxim-methyl, Mancozeb, Mandipropamid, Metalaxyl/mefenoxam, Metiram, Metrafenone, Myclobutanil, Penconazole, Penthiopyrad, Picoxystrobin, Propamocarb, Propiconazole, Propineb, Proquinazid, Prothioconazole, Pyraclostrobin, Pyrimethanil, Quinoxyfen, Spiroxamine, Sulphur, Tebuconazole, Thiophanate-methyl, Trifloxystrobin; Cereals Herbicides: 2.4-D, Amidosulfuron, Bromoxynil, Carfentrazone-E, Chlorotoluron, Chlorsulfuron, Clodinafop-P, Clopyralid, Dicamba, Diclofop-M, Diflufenican, Fenoxaprop, Florasulam, Flucarbazone-NA, Flufenacet, Flupyrosulfuron-M, Fluroxypyr, Flurtamone, Glyphosate, Iodosulfuron, Ioxynil, Isoproturon, MCPA, Mesosulfuron, Metsulfuron, Pendimethalin, Pinoxaden, Propoxycarbazone, Prosulfocarb, Pyroxsulam, Sulfosulfuron, Thifensulfuron, Tralkoxydim, Triasulfuron, Tribenuron, Trifluralin, Tritosulfuron; Cereals Fungicides: Azoxystrobin, Bixafen, Boscalid, Carbendazim, Chlorothalonil, Cyflufenamid, Cyproconazole, Cyprodinil, Dimoxystrobin, Epoxiconazole, Fenpropidin, Fenpropimorph, Fluopyram, Fluoxastrobin, Fluquinconazole, Fluxapyroxad, Isopyrazam, Kresoxim-methyl, Metconazole, Metrafenone, Penthiopyrad, Picoxystrobin, Prochloraz, Propiconazole, Proquinazid, Prothioconazole, Pyraclostrobin, Quinoxyfen, Spiroxamine, Tebuconazole, Thiophanate-methyl, Trifloxystrobin; Cereals Insecticides: Dimethoate, Lambda-cyhalthrin, Deltamethrin, alpha-Cypermethrin, β-cyfluthrin, Bifenthrin, Imidacloprid, Clothianidin, Thiamethoxam, Thiacloprid, Acetamiprid, Dinetofuran, Clorphyriphos, Pirimicarb, Methiocarb, Sulfoxaflor; Maize Herbicides: Atrazine, Alachlor, Bromoxynil, Acetochlor, Dicamba, Clopyralid, (S-)Dimethenamid, Glufosinate, Glyphosate, Isoxaflutole, (S-)Metolachlor, Mesotrione, Nicosulfuron, Primisulfuron, Rimsulfuron, Sulcotrione, Foramsulfuron, Topramezone, Tembotrione, Saflufenacil, Thiencarbazone, Flufenacet, Pyroxasulfon; Maize Insecticides: Carbofuran, Chlorpyrifos, Bifenthrin, Fipronil, Imidacloprid, Lambda-Cyhalothrin, Tefluthrin, Terbufos, Thiamethoxam, Clothianidin, Spiromesifen, Flubendiamide, Triflumuron, Rynaxypyr, Deltamethrin, Thiodicarb, β-Cyfluthrin, Cypermethrin, Bifenthrin, Lufenuron, Tebupirimphos, Ethiprole, Cyazypyr, Thiacloprid, Acetamiprid, Dinetofuran, Avermectin; Maize Fungicides: Azoxystrobin, Bixafen, Boscalid, Cyproconazole, Dimoxystrobin, Epoxiconazole, Fenitropan, Fluopyram, Fluoxastrobin, Fluxapyroxad, Isopyrazam, Metconazole, Penthiopyrad, Picoxystrobin, Propiconazole, Prothioconazole, Pyraclostrobin, Tebuconazole, Trifloxystrobin; Rice Herbicides: Butachlor, Propanil, Azimsulfuron, Bensulfuron, Cyhalofop, Daimuron, Fentrazamide, Imazosulfuron, Mefenacet, Oxaziclomefone, Pyrazosulfuron, Pyributicarb, Quinclorac, Thiobencarb, Indanofan, Flufenacet, Fentrazamide, Halosulfuron, Oxaziclomefone, Benzobicyclon, Pyriftalid, Penoxsulam, Bispyribac, Oxadiargyl, Ethoxysulfuron, Pretilachlor, Mesotrione, Tefuryltrione, Oxadiazone, Fenoxaprop, Pyrimisulfan; Rice Insecticides: Diazinon, Fenobucarb, Benfuracarb, Buprofezin, Dinotefuran, Fipronil, Imidacloprid, Isoprocarb, Thiacloprid, Chromafenozide, Clothianidin, Ethiprole, Flubendiamide, Rynaxypyr, Deltamethrin, Acetamiprid, Thiamethoxam, Cyazypyr, Spinosad, Spinotoram, Emamectin-Benzoate, Cypermethrin, Chlorpyriphos, Etofenprox, Carbofuran, Benfuracarb, Sulfoxaflor; Rice Fungicides: Azoxystrobin, Carbendazim, Carpropamid, Diclocymet, Difenoconazole, Edifenphos, Ferimzone, Gentamycin, Hexaconazole, Hymexazol, Iprobenfos (IBP), Isoprothiolane, Isotianil, Kasugamycin, Mancozeb, Metominostrobin, Orysastrobin, Pencycuron, Probenazole, Propiconazole, Propineb, Pyroquilon, Tebuconazole, Thiophanate-methyl, Tiadinil, Tricyclazole, Trifloxystrobin, Validamycin; Cotton Herbicides: Diuron, Fluometuron, MSMA, Oxyfluorfen, Prometryn, Trifluralin, Carfentrazone, Clethodim, Fluazifop-butyl, Glyphosate, Norflurazon, Pendimethalin, Pyrithiobac-sodium, Trifloxysulfuron, Tepraloxydim, Glufosinate, Flumioxazin, Thidiazuron; Cotton Insecticides: Acephate, Aldicarb, Chlorpyrifos, Cypermethrin, Deltamethrin, Abamectin, Acetamiprid, Emamectin Benzoate, Imidacloprid, Indoxacarb, Lambda-Cyhalothrin, Spinosad, Thiodicarb, Gamma-Cyhalothrin, Spiromesifen, Pyridalyl, Flonicamid Flubendiamide, Triflumuron,Rynaxypyr,Beta-Cyfluthrin,Spirotetramat, Clothianidin, Thiamethoxam, Thiacloprid, Dinetofuran, Flubendiamide, Cyazypyr, Spinosad, Spinotoram, gamma Cyhalothrin, 4-[[(6-Chlorpyridin-3-yl)methyl](2,2-difluorethyl)amino]furan-2(5H)-on, Thiodicarb, Avermectin, Flonicamid, Pyridalyl, Spiromesifen, Sulfoxaflor; Cotton Fungicides: Azoxystrobin, Bixafen, Boscalid, Carbendazim, Chlorothalonil, Copper, Cyproconazole, Difenoconazole, Dimoxystrobin, Epoxiconazole, Fenamidone, Fluazinam, Fluopyram, Fluoxastrobin, Fluxapyroxad, Iprodione, Isopyrazam, Isotianil, Mancozeb, Maneb, Metominostrobin, Penthiopyrad, Picoxystrobin, Propineb, Prothioconazole, Pyraclostrobin, Quintozene, Tebuconazole, Tetraconazole, Thiophanate-methyl, Trifloxystrobin; Soybean Herbicides: Alachlor, Bentazone, Trifluralin, Chlorimuron-Ethyl, Cloransulam-Methyl, Fenoxaprop, Fomesafen, Fluazifop, Glyphosate, Imazamox, Imazaquin, Imazethapyr, (S-)Metolachlor, Metribuzin, Pendimethalin, Tepraloxydim, Glufosinate; Soybean Insecticides: Lambda-cyhalothrin, Methomyl, Imidacloprid, Clothianidin, Thiamethoxam, Thiacloprid, Acetamiprid, Dinetofuran, Flubendiamide, Rynaxypyr, Cyazypyr, Spinosad, Spinotoram, Emamectin-Benzoate, Fipronil, Ethiprole, Deltamethrin, β-Cyfluthrin,gamma and lambda Cyhalothrin, 4-[[(6-Chlorpyridin-3-yl)methyl](2,2-difluorethyl)amino]furan-2(5H)-on, Spirotetramat, Spinodiclofen, Triflumuron, Flonicamid, Thiodicarb, beta-Cyfluthrin; Soybean Fungicides: Azoxystrobin, Bixafen, Boscalid, Carbendazim, Chlorothalonil, Copper, Cyproconazole, Difenoconazole, Dimoxystrobin, Epoxiconazole, Fluazinam, Fluopyram, Fluoxastrobin, Flutriafol, Fluxapyroxad, Isopyrazam, Iprodione, Isotianil, Mancozeb, Maneb, Metconazole, Metominostrobin, Myclobutanil, Penthiopyrad, Picoxystrobin, Propiconazole, Propineb, Prothioconazole, Pyraclostrobin, Tebuconazole, Tetraconazole, Thiophanate-methyl, Trifloxystrobin; Sugarbeet Herbicides: Chloridazon, Desmedipham, Ethofumesate, Phenmedipham, Triallate, Clopyralid, Fluazifop, Lenacil, Metamitron, Quinmerac, Cycloxydim, Triflusulfuron, Tepraloxydim, Quizalofop; Sugarbeet Insecticides: Imidacloprid, Clothianidin, Thiamethoxam, Thiacloprid, Acetamiprid, Dinetofuran, Deltamethrin, β-Cyfluthrin, gamma/lambda Cyhalothrin, 4-[[(6-Chlorpyridin-3-yl)methyl](2,2-difluorethyl)amino]furan-2(5H)-on, Tefluthrin, Rynaxypyr, Cyaxypyr, Fipronil, Carbofuran; Canola Herbicides: Clopyralid, Diclofop, Fluazifop, Glufosinate, Glyphosate, Metazachlor, Trifluralin Ethametsulfuron, Quinmerac, Quizalofop, Clethodim, Tepraloxydim; Canola Fungicides: Azoxystrobin, Bixafen, Boscalid, Carbendazim, Cyproconazole, Difenoconazole, Dimoxystrobin, Epoxiconazole, Fluazinam, Fluopyram, Fluoxastrobin, Flusilazole, Fluxapyroxad, Iprodione, Isopyrazam, Mepiquat-chloride, Metconazole, Metominostrobin, Paclobutrazole, Penthiopyrad., Picoxystrobin, Prochloraz, Prothioconazole, Pyraclostrobin, Tebuconazole, Thiophanate-methyl, Trifloxystrobin, Vinclozolin; Canola Insecticides: Carbofuran, Thiacloprid, Deltamethrin, Imidacloprid, Clothianidin, Thiamethoxam, Acetamiprid, Dinetofuran, β-Cyfluthrin, gamma and lambda Cyhalothrin, tau-Fluvaleriate, Ethiprole, Spinosad, Spinotoram, Flubendiamide, Rynaxypyr, Cyazypyr, 4-[[(6-Chlorpyridin-3-yl)methyl](2,2-difluorethyl)amino]furan-2(5H)-on.

The following examples are offered by way of illustration and not by way of limitation.

### EXPERIMENTAL EXAMPLES

### Example 1. Discovery of novel pesticidal genes from Bacillus thuringiensis

A novel pesticidal gene was identified from bacterial strain ATX47290 (Table 1).

**Table 1. Novel gene identified from strain ATX47290**

| **Gene name** | **Molecular weigh**t **(kD)** | **Closest homolog** | **Nucleotide SEQ ID NO** | **Amino acid SEQ ID NO** |
|---|---|---|---|---|
| Axmi277 | 133.1 | 73.5% Axmi031 | 1 | 2 |
| Axmi277(trun) | | 74.4% Cry14Aa (trun) | | 3 |

Axmi277 is amplified by PCR from pAX980, and the PCR product is cloned into the *Bacillus* expression vector pAX916, or another suitable vector, by methods well known in the art. The resulting *Bacillus* strain, containing the vector with *axmi* gene is cultured on a conventional growth media, such as CYS media (10 g/l Bacto-casitone; 3 g/l yeast extract; 6 g/l KH₂PO₄; 14 g/l K₂HPO₄; 0.5 mM MgSO₄; 0.05 mM MnCl₂; 0.05 mM FeSO₄), until sporulation is evident by microscopic examination. Samples are prepared and tested for activity in bioassays.

### Example 2. Activity of Axmi277 against C. elegans

The full-length and truncated genes were overexpressed in *E. coli* BL21(*DE3*) star strain behind the leader sequence that encodes for maltose binding protein (pMAL expression vector). The cultures were grown at 37°C, 250 rpm in LB medium until the cultures reached an optical density (OD600) of 0.4 to 0.6, then cultures were switched to 20°C and protein production was induced by addition of 0.2 to 0.3 mM IPTG. The cells were harvested after 16 hours and the cell pellet was stored for protein purification. The heterologous overexpressed proteins were purified using an AKTA FPLC system by affinity chromatography using amylose/agarose beads. The resin was used for isolation of proteins fused to maltose binding proteins. The proteins were eluted out of the column in the first 1 to 2 column volumes with buffer containing 10mM maltose. The purity and characterization of the proteins were done by SDS-PAGE analysis.

Axmi277 showed very strong activity against *C*. *elegans* (80% average mortality compared with controls). Both forms of the proteins, full length and truncated version, showed strong activity at low concentration, and the activities were heat labile. Before submission to bioassays, the proteins were processed with Factor Xa for removal of MBP fragment.

### Example 3. Additional Assays for Pesticidal Activity

The nucleotide sequences of the invention can be tested for their ability to produce pesticidal proteins. The ability of a pesticidal protein to act as a pesticide upon a pest is often assessed in a number of ways. One way well known in the art is to perform a feeding assay. In such a feeding assay, one exposes the pest to a sample containing either compounds to be tested or control samples. Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, and then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouth parts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson and Preisler, eds. (1992) Pesticide bioassays with arthropods, CRC, Boca Raton, FL. Alternatively, assays are commonly described in the journals Arthropod Management Tests and Journal of Economic Entomology or by discussion with members of the Entomological Society of America (ESA).

In some embodiments, the DNA regions encoding the toxin region of the pesticidal proteins disclosed herein are cloned into the *E. coli* expression vector pMAL-C4x behind the *malE* gene coding for Maltose binding protein (MBP). These in-frame fusions result in MBP-Axmi fusion proteins expression in *E. coli.*

For expression in *E. coli,* BL21*DE3 are transformed with individual plasmids. Single colonies are inoculated in LB supplemented with carbenicillin and glucose, and grown overnight at 37°C. The following day, fresh medium is inoculated with 1% of overnight culture and grown at 37°C to logarithmic phase. Subsequently, cultures are induced with 0.3mM IPTG overnight at 20°C. Each cell pellet is suspended in 20mM Tris-Cl buffer, pH 7.4 + 200mM NaCl + 1mM DTT + protease inhibitors and sonicated. Analysis by SDS-PAGE can be used to confirm expression of the fusion proteins.

Total cell free extracts are then run over amylose column attached to fast protein liquid chromatography (FPLC) for affinity purification of MBP-axmi fusion proteins. Bound fusion proteins are eluted from the resin with 10mM maltose solution. Purified fusion proteins are then cleaved with either Factor Xa or trypsin to remove the amino terminal MBP tag from the Axmi protein. Cleavage and solubility of the proteins can be determined by SDS-PAGE

### Example 4. Vectoring of Genes for Plant Expression

The coding regions of the invention are connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

In one aspect of the invention, synthetic DNA sequences are designed and generated. These synthetic sequences have altered nucleotide sequence relative to the parent sequence, but encode proteins that are essentially identical to the parent sequence.

In another aspect of the invention, modified versions of the synthetic genes are designed such that the resulting peptide is targeted to a plant organelle, such as the endoplasmic reticulum or the apoplast. Peptide sequences known to result in targeting of fusion proteins to plant organelles are known in the art. For example, the N-terminal region of the acid phosphatase gene from the White Lupin *Lupinus albus* (GENBANK® ID GI: 14276838, Miller et al. (2001) Plant Physiology 127: 594-606) is known in the art to result in endoplasmic reticulum targeting of heterologous proteins. If the resulting fusion protein also contains an endoplasmic reticulum retention sequence comprising the peptide N-terminus-lysine-aspartic acid-glutamic acid-leucine (i.e., the "KDEL" motif, SEQ ID NO:4) at the C-terminus, the fusion protein will be targeted to the endoplasmic reticulum. If the fusion protein lacks an endoplasmic reticulum targeting sequence at the C-terminus, the protein will be targeted to the endoplasmic reticulum, but will ultimately be sequestered in the apoplast.

Thus, this gene encodes a fusion protein that contains the N-terminal thirty-one amino acids of the acid phosphatase gene from the White Lupin *Lupinus albus* (GENBANK® ID GI:14276838, Miller *et al.,* 2001, *supra)* fused to the N-terminus of the amino acid sequence of the invention, as well as the KDEL sequence at the C-terminus. Thus, the resulting protein is predicted to be targeted the plant endoplasmic reticulum upon expression in a plant cell.

The plant expression cassettes described above are combined with an appropriate plant selectable marker to aid in the selection of transformed cells and tissues, and ligated into plant transformation vectors. These may include binary vectors from *Agrobacterium*-mediated transformation or simple plasmid vectors for aerosol or biolistic transformation.

### Example 5. Transformation of Maize Cells with the pesticidal protein genes described herein

Maize ears are best collected 8-12 days after pollination. Embryos are isolated from the ears, and those embryos 0.8-1.5 mm in size are preferred for use in transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 mL/L (of 1 mg/mL Stock) 2,4-D). However, media and salts other than DN62A5S are suitable and are known in the art. Embryos are incubated overnight at 25°C in the dark. However, it is not necessary *per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to the genes of the invention in plant cells are accelerated into plant tissue using an aerosol beam accelerator, using conditions essentially as described in PCT Publication No. WO/0138514. After beaming, embryos are incubated for about 30 min on osmotic media, and placed onto incubation media overnight at 25°C in the dark. To avoid unduly damaging beamed explants, they are incubated for at least 24 hours prior to transfer to recovery media. Embryos are then spread onto recovery period media, for about 5 days, 25°C in the dark, then transferred to a selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

### DN62A5S Media

| Components | Per Liter | Source |
|---|---|---|
| Chu's N6 Basal Salt Mixture (Prod. No. C 416) | 3.98 g/L | Phytotechnology Labs |
| Chu's N6 Vitamin Solution (Prod. No. C 149) | 1 mL/L (of 1000x Stock) | Phytotechnology Labs |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

The pH of the solution is adjusted to pH 5.8 with 1N KOH/1N KCl, Gelrite (Sigma) is added at a concentration up to 3g/L, and the media is autoclaved. After cooling to 50°C, 2 ml/L of a 5 mg/ml stock solution of silver nitrate (Phytotechnology Labs) is added.

### Example 6. Transformation of genes of the invention in Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art.

## Claims

1. A recombinant nucleic acid molecule comprising a nucleotide sequence encoding an amino acid sequence having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3.

2. The recombinant nucleic acid molecule of claim 1, wherein:
(a) said nucleotide sequence is a synthetic sequence that has been designed for expression in a plant; or
(b) said nucleotide sequence is operably linked to a promoter capable of directing expression of said nucleotide sequence in a plant cell.

3. A vector comprising the recombinant nucleic acid molecule of claim 1, preferably further comprising a nucleic acid molecule encoding a heterologous polypeptide.

4. A host cell that contains the recombinant nucleic acid of claim 1, preferably that is a bacterial host cell or a plant cell.

5. A transgenic plant comprising the plant host cell of claim 4, preferably wherein said plant is selected from the group consisting of maize, sorghum, wheat, cabbage, sunflower, tomato, crucifers, peppers, potato, cotton, rice, soybean, sugarbeet, sugarcane, tobacco, barley, and oilseed rape.

6. A transgenic seed comprising the nucleic acid molecule of claim 1.

7. A recombinant polypeptide with nematocidal activity, selected from the group consisting of:
a) a polypeptide comprising the amino acid sequence of any of SEQ ID NO:2 or 3; and
b) a polypeptide comprising an amino acid sequence having at least 95% sequence identity to the amino acid sequence of any of SEQ ID NO:2 or 3.

8. The polypeptide of claim 7 further comprising heterologous amino acid sequences.

9. A composition comprising the polypeptide of claim 7.

10. The composition of claim 9, wherein:
(a) said composition is selected from the group consisting of a powder, dust, pellet, granule, spray, emulsion, colloid, and solution;
(b) said composition is prepared by desiccation, lyophilization, homogenization, extraction, filtration, centrifugation, sedimentation, or concentration of a culture of bacterial cells; or
(c) said composition comprises from about 1% to about 99% by weight of said polypeptide.

11. A method for controlling a nematode pest population or for killing a nematode pest, comprising contacting said population or pest with a pesticidally-effective amount of the polypeptide of claim 7.

12. A method for producing a polypeptide with nematocidal activity, comprising culturing the host cell of claim 4 under conditions in which the nucleic acid molecule encoding the polypeptide is expressed.

13. A plant having stably incorporated into its genome a DNA construct comprising a nucleotide sequence that encodes a protein having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3, preferably wherein said plant is a plant cell.

14. A method for protecting a plant from a pest, comprising expressing in a plant or cell thereof a nucleotide sequence that encodes a nematocidal polypeptide, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3.

15. A method for increasing yield in a plant comprising growing in a field a plant of or a seed thereof having stably incorporated into its genome a DNA construct comprising a nucleotide sequence that encodes a protein having nematocidal activity, wherein said nucleotide sequence is selected from the group consisting of:
a) the nucleotide sequence set forth in SEQ ID NO:1;
b) a nucleotide sequence that encodes a polypeptide comprising the amino acid sequence of any of SEQ ID NO:2 or 3; and
c) a nucleotide sequence that encodes a polypeptide comprising an amino acid sequence having at least 95% sequence identity to the amino acid sequence of any of SEQ ID NO:2 or 3;
wherein said field is infested with a nematode against which said polypeptide has nematocidal activity.

## Patentansprüche

1. Rekombinantes Nukleinsäuremolekül, umfassend eine Nukleotidsequenz, die eine Aminosäuresequenz mit nematizider Aktivität kodiert, wobei die Nukleotidsequenz ausgewählt ist aus der Gruppe bestehend aus:
a) der in SEQ ID NO: 1 dargestellten Nukleotidsequenz;
b) einer Nukleotidsequenz, die ein Polypeptid kodiert, das die Aminosäuresequenz einer SEQ ID NO: 2 oder 3 umfasst; und
c) einer Nukleotidsequenz, die ein Polypeptid kodiert, das eine Aminosäuresequenz mit mindestens 95 % Sequenzidentität zu der Aminosäuresequenz einer von SEQ ID NO: 2 oder 3 umfasst.

2. Rekombinantes Nukleinsäuremolekül nach Anspruch 1, wobei:
(a) die Nukleotidsequenz eine synthetische Sequenz ist, die zur Expression in einer Pflanze konzipiert wurde; oder
(b) die Nukleotidsequenz funktionsfähig mit einem Promotor verbunden ist, der in der Lage ist, eine Expression der Nukleotidsequenz in einer Pflanzenzelle zu lenken.

3. Vektor, umfassend das rekombinante Nukleinsäuremolekül nach Anspruch 1, vorzugsweise ferner umfassend ein Nukleinsäuremolekül, das ein heterologes Polypeptid kodiert.

4. Wirtszelle, die die rekombinante Nukleinsäure nach Anspruch 1 enthält, die vorzugsweise eine bakterielle Wirtszelle oder eine Pflanzenzelle ist.

5. Transgene Pflanze, umfassend die Pflanzenwirtszelle nach Anspruch 4, vorzugsweise wobei die Pflanze ausgewählt ist aus der Gruppe bestehend aus Mais, Sorghum, Weizen, Kohl, Sonnenblume, Tomate, Kreuzblütlern, Paprika, Kartoffel, Baumwolle, Reis, Soja, Zuckerrübe, Zuckerrohr, Tabak, Gerste und Raps.

6. Transgener Samen, umfassend das Nukleinsäuremolekül nach Anspruch 1.

7. Rekombinantes Polypeptid mit nematizider Aktivität, ausgewählt aus der Gruppe bestehend aus:
a) einem Polypeptid, umfassend die Aminosäuresequenz einer von SEQ ID NO: 2 oder 3; und
b) einem Polypeptid, umfassend eine Aminosäuresequenz mit mindestens 95 % Sequenzidentität zu der Aminosäuresequenz einer von SEQ ID NO: 2 oder 3.

8. Polypeptid nach Anspruch 7, ferner umfassend heterologe Aminosäuresequenzen.

9. Zusammensetzung, umfassend das Polypeptid nach Anspruch 7.

10. Zusammensetzung nach Anspruch 9, wobei:
(a) die Zusammensetzung ausgewählt ist aus der Gruppe bestehend aus einem Pulver, Staub, Pellet, Granulat, Spray, Emulsion, Kolloid und Lösung;
(b) die Zusammensetzung durch Trocknung, Lyophilisierung, Homogenisierung, Extraktion, Filtration, Zentrifugation, Sedimentation oder Konzentration einer Kultur von Bakterienzellen hergestellt wird; oder
(c) die Zusammensetzung etwa 1 Gewichtsprozent bis etwa 99 Gewichtsprozent des Polypeptids umfasst.

11. Verfahren zum Steuern einer Nematodenschädlingspopulation oder zum Abtöten eines Nematodenschädlings, umfassend ein Inkontaktbringen der Population oder des Schädlings mit einer pestizidwirksamen Menge des Polypeptids nach Anspruch 7.

12. Verfahren zum Herstellen eines Polypeptids mit nematizider Aktivität, umfassend
Kultivieren der Wirtszelle nach Anspruch 4 unter Bedingungen, bei denen das Nukleinsäuremolekül exprimiert wird, das das Polypeptid kodiert.

13. Pflanze, die ein DNA-Konstrukt aufweist, das stabil in ihr Genom integriert ist, das eine Nukleotidsequenz umfasst, die ein Protein mit nematizider Aktivität kodiert, wobei die Nukleotidsequenz ausgewählt ist aus der Gruppe bestehend aus:
a) der in SEQ ID NO: 1 dargestellten Nukleotidsequenz;
b) einer Nukleotidsequenz, die ein Polypeptid kodiert, das die Aminosäuresequenz einer SEQ ID NO: 2 oder 3 umfasst; und
c) einer Nukleotidsequenz, die ein Polypeptid kodiert, das eine Aminosäuresequenz mit mindestens 95 % Sequenzidentität zu der Aminosäuresequenz einer von SEQ ID NO: 2 oder 3 umfasst, vorzugsweise wobei die Pflanze eine Pflanzenzelle ist.

14. Verfahren zum Schützen einer Pflanze vor einem Schädling, umfassend ein Exprimieren einer Nukleotidsequenz, die ein nematizides Polypeptid kodiert, in einer Pflanze oder Zelle davon, wobei die Nukleotidsequenz ausgewählt ist aus der Gruppe bestehend aus:
a) der Nukleotidsequenz gemäß SEQ ID NO: 1;
b) einer Nukleotidsequenz, die ein Polypeptid kodiert, das die Aminosäuresequenz einer SEQ ID NO: 2 oder 3 umfasst; und
c) einer Nukleotidsequenz, die ein Polypeptid kodiert, das eine Aminosäuresequenz mit mindestens 95 % Sequenzidentität zu der Aminosäuresequenz einer von SEQ ID NO: 2 oder 3 umfasst.

15. Verfahren zum Erhöhung eines Ertrag in einer Pflanze, umfassend ein Züchten einer Pflanze oder eines Samens davon in einem Feld, in deren/dessen Genom ein DNA-Konstrukt mit einer Nukleotidsequenz, die für ein Protein mit nematozider Aktivität kodiert, stabil integriert ist, wobei die Nukleotidsequenz ausgewählt ist aus der Gruppe bestehend aus:
a) der in SEQ ID NO: 1 dargestellten Nukleotidsequenz;
b) einer Nukleotidsequenz, die ein Polypeptid kodiert, das die Aminosäuresequenz einer SEQ ID NO: 2 oder 3 umfasst; und
c) einer Nukleotidsequenz, die ein Polypeptid kodiert, das eine Aminosäuresequenz mit mindestens 95 % Sequenzidentität zu der Aminosäuresequenz einer von SEQ ID NO: 2 oder 3 umfasst;
wobei das Feld mit einem Nematoden befallen ist, gegen den das Polypeptid eine nematizide Aktivität aufweist.

## Revendications

1. Molécule d'acide nucléique recombinant comprenant une séquence nucléotidique codant pour une séquence d'acides aminés ayant une activité nématocide, ladite séquence nucléotidique étant choisie dans le groupe constitué par :
a) la séquence nucléotidique indiquée dans SEQ ID N° : 1 ;
b) une séquence nucléotidique qui code pour un polypeptide comprenant la séquence d'acides aminés de l'une quelconque de SEQ ID N° : 2 ou 3 ; et
c) une séquence nucléotidique qui code pour un polypeptide comprenant une séquence d'acides aminés ayant au moins 95 % d'identité de séquence avec la séquence d'acides aminés de l'une quelconque de SEQ ID N° : 2 ou 3.

2. Molécule d'acide nucléique recombinant de la revendication 1, dans laquelle :
(a) ladite séquence nucléotidique est une séquence de synthèse qui a été conçue pour une expression dans un végétal ; ou
(b) ladite séquence nucléotidique est fonctionnellement liée à un promoteur capable d'orienter l'expression de ladite séquence nucléotidique dans une cellule végétale.

3. Vecteur comprenant la molécule d'acide nucléique recombinant de la revendication 1, comprenant en outre de préférence une molécule d'acide nucléique codant pour un polypeptide hétérologue.

4. Cellule hôte qui contient l'acide nucléique recombinant de la revendication 1, qui est de préférence une cellule hôte bactérienne ou une cellule végétale.

5. Végétal transgénique comprenant la cellule hôte végétale de la revendication 4, ledit végétale transgénique étant de préférence choisi dans le groupe constitué par le maïs, le sorgho, le blé, le chou, le tournesol, la tomate, les brassicacées, les poivrons, la pomme de terre, le coton, le riz, le soja, la betterave, la canne à sucre, le tabac, l'orge et le colza oléagineux.

6. Semence transgénique comprenant la molécule d'acide nucléique de la revendication 1.

7. Polypeptide recombinant ayant une activité nématocide, choisi dans le groupe constitué par :
b) un polypeptide comprenant la séquence d'acides aminés de l'une quelconque de SEQ ID N° : 2 ou 3 ; et
c) un polypeptide comprenant une séquence d'acides aminés ayant au moins 95 % d'identité de séquence avec la séquence d'acides aminés de l'une quelconque de SEQ ID N° : 2 ou 3.

8. Polypeptide de la revendication 7, comprenant en outre des séquences d'acides aminés hétérologues.

9. Composition comprenant le polypeptide de la revendication 7.

10. Composition de la revendication 9, dans laquelle :
(a) ladite composition est choisi dans le groupe constitué par une poudre, une poussière, une pastille, une granulé, une pulvérisation, une émulsion, un colloïde et une solution ;
(b) ladite composition est préparée par dessiccation, lyophilisation, homogénéisation, extraction, filtration, centrifugation, sédimentation ou concentration d'une culture de cellules bactériennes ; ou
(c) ladite composition comprend environ 1 % à environ 99 % en poids dudit polypeptide.

11. Procédé de lutte contre une population de nématodes nuisibles ou d'élimination d'un nématode nuisible, comprenant la mise en contact de ladite population ou dudit nuisible avec une quantité pesticide efficace du polypeptide de la revendication 7.

12. Procédé de production d'un polypeptide ayant une activité nématocide, comprenant la mise en culture de la cellule hôte de la revendication 4 dans des conditions dans lesquelles la molécule d'acide nucléique codant pour le polypeptide est exprimée.

13. Végétal dans le génome duquel est incorporé de manière stable un produit de construction d'ADN comprenant une séquence nucléotidique qui code pour une protéine ayant une activité nématocide, ladite séquence nucléotidique étant choisie dans le groupe constitué par :
a) la séquence nucléotidique indiquée dans SEQ ID N° : 1 ;
b) une séquence nucléotidique qui code pour un polypeptide comprenant la séquence d'acides aminés de l'une quelconque de SEQ ID N° : 2 ou 3 ; et
c) une séquence nucléotidique qui code pour un polypeptide comprenant une séquence d'acides aminés ayant au moins 95 % d'identité de séquence avec la séquence d'acides aminés de l'une quelconque de SEQ ID N° : 2 ou 3, ledit végétal étant de préférence une cellule végétale.

14. Procédé de protection d'un végétale contre un nuisible, comprenant l'expression dans un végétal ou une cellule de celui-ci d'une séquence nucléotidique qui code pour un polypeptide nématocide, ladite séquence nucléotidique étant choisie dans le groupe constitué par :
a) la séquence nucléotidique indiquée dans SEQ ID N° : 1 ;
b) une séquence nucléotidique qui code pour un polypeptide comprenant la séquence d'acides aminés de l'une quelconque de SEQ ID N° : 2 ou 3 ; et
c) une séquence nucléotidique qui code pour un polypeptide comprenant une séquence d'acides aminés ayant au moins 95 % d'identité de séquence avec la séquence d'acides aminés de l'une quelconque de SEQ ID N° : 2 ou 3.

15. Procédé d'accroissement du rendement d'un végétal, comprenant la croissance dans un champ d'un végétal ou d'une semence de celui-ci dans le génome duquel est incorporé de manière stable un produit de construction d'ADN comprenant une séquence nucléotidique codant pour une protéine ayant une activité nématocide, ladite séquence nucléotidique étant choisie dans le groupe constitué par :
a) la séquence nucléotidique indiquée dans SEQ ID N° : 1 ;
b) une séquence nucléotidique qui code pour un polypeptide comprenant la séquence d'acides aminés de l'une quelconque de SEQ ID N° : 2 ou 3 ; et
c) une séquence nucléotidique qui code pour un polypeptide comprenant une séquence d'acides aminés ayant au moins 95 % d'identité de séquence avec la séquence d'acides aminés de l'une quelconque de SEQ ID N° : 2 ou 3 ;
ledit champ étant infesté par un nématode contre lequel ledit polypeptide exerce une activité nématocide.
